# EUROPEAN PATENT SPECIFICATION

(11) **EP 2 773 611 B1**
(45) Date of publication and mention of the grant of the patent: **11.01.2017**
(21) Application number: 12845573.0
(22) Date of filing: 31.10.2012
(51) Int. Cl.: C07C 231/18, C07C 233/47, C07B 53/00, C07B 61/00, C07F 15/00, C07C 67/31, C07C 231/12

(54) **METHOD FOR PRODUCING OPTICALLY ACTIVE -HYDROXY- -AMINOCARBOXYLIC ACID ESTER**
VERFAHREN ZUR HERSTELLUNG OPTISCH AKTIVER HYDROXY-AMINOCARBONSÄUREESTER
PROCÉDÉ DE PRODUCTION D'UN ESTER D'ACIDE -HYDROXY- -AMINOCARBOXYLIQUE OPTIQUEMENT ACTIF

(30) Priority: 31.10.2011 JP 2011239283
(43) Date of publication of application: 10.09.2014
(73) Proprietor: Takasago International Corporation, Tokyo 144-8721 (JP)
(72) Inventor: TANAKA, Shigeru, Hiratsuka-shi Kanagawa 254-0073 (JP); TOUGE, Taichiro, Hiratsuka-shi Kanagawa 254-0073 (JP); NARA, Hideki, Hiratsuka-shi Kanagawa 254-0073 (JP); ISHIDA, Kenya, Tokyo 1448721 (JP)
(74) Representative: Zimmermann & Partner Patentanwälte mbB
(86) International application number: PCT/JP2012/078771
(87) International publication number: WO 2013/065867

(56) References cited:
- EP-A1- 2 067 769
- WO-A1-2008/041571
- WO-A1-2012/147944
- WO-A2-2010/106364
- JP-A- 2012 067 071
- TAICHIRO TOUGE ET AL.: 'Oxo-Tethered Ruthenium(III) Complex as a Bifunctional Catalyst for Asymmetric Transfer Hydrogenation and H2 Hydrogenation' JOURNAL OF THE AMERICAN CHEMICAL SOCIETY vol. 133, no. 38, 26 August 2011, pages 14960 - 14963, XP009152861
- Takao Ikariya ET AL: "Asymmetric Transfer Hydrogenation of Ketones with Bifunctional Transition Metal-Based Molecular Catalysts +", ACCOUNTS OF CHEMICAL RESEARCH., vol. 40, no. 12, 1 December 2007 (2007-12-01), pages 1300-1308, XP055280521, US ISSN: 0001-4842, DOI: 10.1021/ar700134q

## Description

### Technical Field

The present invention relates to a novel method for producing an optically active ß-hydroxy-α-aminocarboxylic acid ester, the method comprising performing an asymmetric reduction reaction of a ß-keto-α-aminocarboxylic acid ester by use of a ruthenium complex as catalyst.

### Background Art

Optically active β-hydroxy-α-aminocarboxylic acid esters are important as synthetic intermediates of ceramides, which are key molecules of the moisturizing effect on the stratum corneum, and are compounds which can serve as not only intermediates of pharmaceuticals and the like, but also important intermediates for producing functional materials and the like necessary in the chemical industries and the like. For this reason, methods for producing an optically active ß-hydroxy-α-aminocarboxylic acid ester have been studied and reported so far (see JP-H06-080617A or WO2008/041571A).

JP-H06-080617A discloses a method in which a syn isomer of an optically active β-hydroxy-α-aminocarboxylic acid ester is selectively synthesized by a catalytic asymmetric hydrogenation reaction using a ruthenium-optically active phosphine complex, and further the hydroxyl group at the ß-position is optically inverted to obtain the anti isomer.

WO2008/041571A discloses a method in which an anti isomer is selectively obtained by conducting a catalytic asymmetric hydrogen transfer reaction using a ruthenium-optically active diamine complex.

Meanwhile, many asymmetric reactions including asymmetric reduction have been developed, and many asymmetric reactions have been reported in which an asymmetric metal complex having an optically active phosphine ligand is used. Moreover, for example, many reports indicate that complexes in which an optically active nitrogen compound is coordinated to a transition metal such as ruthenium, rhodium, or iridium have excellent performances as catalysts for asymmetric synthesis reactions (see Chem Rev., (1992), p. 1051; J. Am. Chem. Soc., 117 (1995), p. 7562; J. Am. Chem. Soc., 118 (1996), p. 2521; and J. Am. Chem. Soc., 118 (1996), p. 4916).

WO 2010/106364 A2 describes a process for hydrogenating a substrate comprising a carbon-heteroatom double bond. The process comprises the step of reacting the substrate with hydrogen gas in the presence of a hydrogenation catalyst, wherein the hydrogenation catalyst is a complex of formula (I).

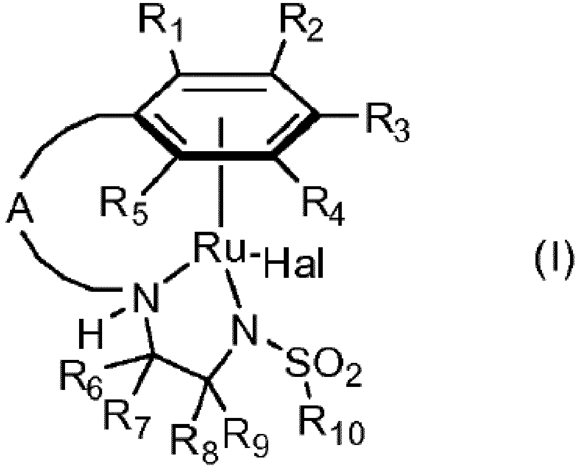

Taichiro Touge et al., Oxo-Tethered Ruthenium(III)Complex as a Bifunctional Catalyst for Asymmetric Transfer Hydrogenation and H2 Hydrogenation, Journal of the American Chemical Society, Vol. 133, No. 38, 26 August 2011, pages 14960 to 14963, describe oxo-tethered Ru amido complexes (*R,R*)-1 and their HCl adducts (*R,R*)-2 exhibited excellent catalytic performance for both asymmetric transfer hydrogenation and the hydrogenation of ketonic substrates under neutral conditions without any co-catalysts to give chiral secondary alcohols with high levels of enatioselectivity.

### Summary of Invention

However, optically active β-hydroxy-α-aminocarboxylic acid esters are obtained only after many steps in the production of the optically active β-hydroxy-α-aminocarboxylic acid esters based on any of the methods reported so far. In addition, even when the ester can be produced in one step by utilizing an asymmetric reduction reaction, the production has such problems that long reaction time is necessary, that the stereo selectivity is low, and that the large amount of the catalyst necessary for the reaction complicates the operation for removing the catalyst. Specifically, the method disclosed in Patent Literature 1 requires an extra step, because a syn isomer is first obtained selectively, and the hydroxyl group at the β-position has to be optically inverted to obtain an anti isomer. In addition, although the anti isomer can be obtained selectively in the method disclosed in Patent Literature 2, the time required for the reaction in this method is as long as several days, and if the reaction time is shortened, the amount of the catalyst is increased. Hence, the method is disadvantageous from the industrial viewpoint.

An object of the present invention is to solve these problems.

To solve the above-described problems, the present inventors have conducted earnest study. As a result, the present inventors have found that the use of the following ruthenium complex as a catalyst makes it possible to efficiently produce an optically active β-hydroxy-α-aminocarboxylic acid ester by an asymmetric reduction of a β-keto-α-aminocarboxylic acid ester under a mild conditions in an anti isomer-selective manner. Specifically, the ruthenium complex is such that the aromatic compound (arene) moiety is coordinated to a ruthenium atom, and that a hetero atom such as an oxygen atom or a sulfur atom is introduced to a chain moiety linking the aromatic compound (arene) moiety and a diamine moiety, or the aromatic compound (arene) moiety and the diamine moiety are is linked with each other through a carbon chain. Moreover, the ruthenium complex has a tridentate ligand whose two nitrogen atoms in the diamine ligand are bonded to the ruthenium atom by covalent bonding or coordination bonding, and whose aromatic compound (arene) moiety linked to the diamine is also coordinated to the ruthenium atom. In addition, in the ruthenium complex, a hetero atom such as an oxygen atom or a sulfur atom is introduced to the chain moiety linking the aromatic compound (arene) moiety and the diamine moiety, or the chain moiety linking the aromatic compound (arene) moiety and the diamine moiety are linked with each other through a carbon chain. This finding has led to the completion of the present invention.

Specifically, the present invention encompasses the following content.
[1] A method for producing an optically active β-hydroxy-α-aminocarboxylic acid ester, comprising performing an asymmetric reduction reaction of a β-keto-α-aminocarboxylic acid ester in the presence of a ruthenium complex and a hydrogen donor, wherein
   the ruthenium complex is represented by the following general formula (1) or (1)': 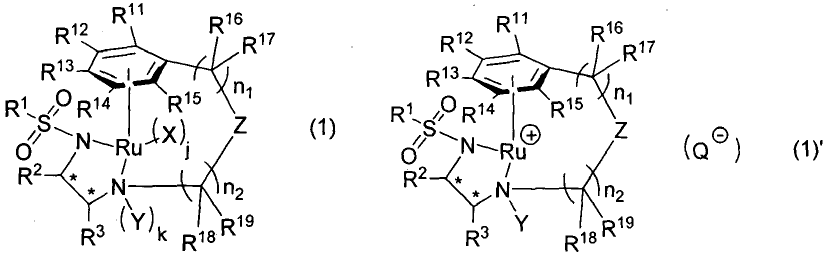 (where
   R¹ represents an alkyl group having 1 to 10 carbon atoms; a halogenated alkyl group having 1 to 10 carbon atoms; a 10-camphoryl group; an amino group which may be substituted with one or two alkyl groups having 1 to 10 carbon atoms; or an aryl group (provided that the aryl group may be substituted with one or more selected from alkyl groups having 1 to 10 carbon atoms, halogenated alkyl groups having 1 to 10 carbon atoms, halogen atoms, cyano groups (-CN), amino groups, alkylamino groups (-NR²⁰R²¹), 5- or 6-membered cyclic amino groups, acylamino groups (-NH-CO-R²⁰), hydroxyl groups, alkoxy groups (-OR²⁰), acyl groups (-CO-R²⁰), carboxyl groups, alkoxycarbonyl groups (-COOR²⁰), phenoxycarbonyl groups, mercapto groups, alkylthio groups (-SR²⁰), silyl groups (-SiR²⁰R²¹R²²), and nitro groups (-NO₂)),
   R²⁰, R²¹, and R²² each independently represent a hydrogen atom, an alkyl group having 1 to 10 carbon atoms, or a cycloalkyl group having 3 to 10 carbon atoms,
   Y represents a hydrogen atom,
   X represents a trifluoromethanesulfonyloxy group, a p-toluenesulfonyloxy group, a methanesulfonyloxy group, a benzenesulfonyloxy group, a hydrogen atom, or a halogen atom,
   Q_{Θ} represents a counter anion,
   j and k each represent 0 or 1, provided that cases where j+k=1 are excluded,
   R² and R³ each independently represent a hydrogen atom; an alkyl group having 1 to 10 carbon atoms; a phenyl group (provided that the phenyl group may be substituted with one or more selected from alkyl groups having 1 to 10 carbon atoms, alkoxy groups having 1 to 10 carbon atoms, and halogen atoms); or a cycloalkyl group having 3 to 8 carbon atoms, or R² and R³ may together form a ring,
   R¹¹, R¹², R¹³, R¹⁴, and R¹⁵ each independently represent a hydrogen atom, an alkyl group having 1 to 10 carbon atoms, or an alkoxy group having 1 to 10 carbon atoms,
   R¹⁶, R¹⁷ R¹⁸, and R¹⁹ each independently represent a hydrogen atom, a hydroxyl group, an alkyl group having 1 to 10 carbon atoms, or an alkoxy group having 1 to 10 carbon atoms, or R¹⁶, R¹⁷, and the carbon atom to which R¹⁶ and R¹⁷ are bonded and/or R¹⁸, R¹⁹, and the carbon atom to which R¹⁸ and R¹⁹ are bonded may form a carbonyl group(s),
   Z represents an oxygen atom, a sulfur atom, or a methylene, n₁ represents 1 or 2, and n₂ represents any integer of 1 to 3, and
   each * indicates an asymmetric carbon atom (provided that when R² and/or R³ is/are a hydrogen atom(s), the carbon atom to which the hydrogen atom is bonded is not an asymmetric carbon atom)),
   the ß-keto-α-aminocarboxylic acid ester is represented by the following general formula (2): 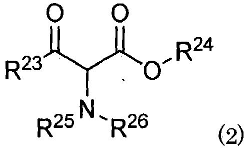 (where
   R²³ represents a hydrocarbon group which has 11 to 21 carbon atoms and which may be substituted with one or more hydroxyl groups,
   R²⁴ represents a hydrogen atom or a hydrocarbon group having 1 to 10 carbon atoms, and
   R²⁵ and R²⁶, which may be the same or different, each represent a hydrogen atom, an alkyl group which has 1 to 10 carbon atoms and which may be substituted with one or more selected from halogen atoms and hydroxyl groups, an acyl group which has 1 to 24 carbon atoms and which may be substituted with one or more selected from halogen atoms and hydroxyl groups, or an amino-protecting group, or R²⁵ and R²⁶ may form, together with the adjacent nitrogen atom, a heterocycle which may be substituted with one or more hydroxyl groups), and
   the optically active β-hydroxy-α-aminocarboxylic acid ester is represented by the following general formula (3) or (4): 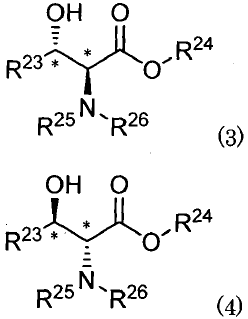 (where
   each * indicates an asymmetric carbon atom, and
   R²³ R²⁴, R²⁵, and R²⁶ are the same as those described above).

In a preferable embodiment, a major product of the optically active β-hydroxy-α-aminocarboxylic acid ester is a (2R,3R) isomer, represented by general formula (4),
the formation ratio (2R,3R) isomer:(2S,3R) isomer is 85:15 to 100:0; and
the reaction is completed within a reaction time of 10 hours.

In another preferable embodiment, a major product of the optically active β-hydroxy-α-aminocarboxylic acid ester is a (2R,3R) isomer represented by general formula (4),
the formation ratio (2R,3R) isomer:(2S,3R) isomer is 85:15 to 100:0; and
the reaction is completed within a reaction time of 20 hours when the molar ratio of the ruthenium complex represented by general formula (1) to the β-keto-α-aminocarboxylic acid ester represented by general formula (2) is 1/500 to 1/10000.

The present invention provides a method for producing an optically active β-hydroxy-α-aminocarboxylic acid ester, which is important as a synthetic intermediate of ceramides, pharmaceuticals, agricultural chemicals, and the like, through simple steps and at low costs.

The ruthenium complex of the present invention has a hetero atom introduced to the chain moiety linking the aromatic compound (arene) moiety and the diamine moiety coordinated to the ruthenium, has an extremely high catalytic activity, and is useful as a catalyst for various kinds of hydrogenation such as reduction of ester groups. Moreover, the ruthenium complex of the present invention has an optically active ligand, and hence is capable of achieving an excellent stereo selectivity and a high enantiomeric excess. The use of this ruthenium complex of the present invention in the method for producing an optically active β-hydroxy-α-aminocarboxylic acid ester makes it possible to synthesize an anti isomer of an optically active β-hydroxy-α-aminocarboxylic acid ester at a high optical purity and in a high yield more simply and efficiently than conventional cases.

### Description of Embodiments

Hereinafter, the present invention will be described in further detail.

In the present invention, an optically active β-hydroxy-α-aminocarboxylic acid ester is produced by performing an asymmetric reduction reaction of a β-keto-α-aminocarboxylic acid ester in the presence of a ruthenium complex and a hydrogen donor, wherein
the ruthenium complex is represented by the following general formula (1) or (1)': 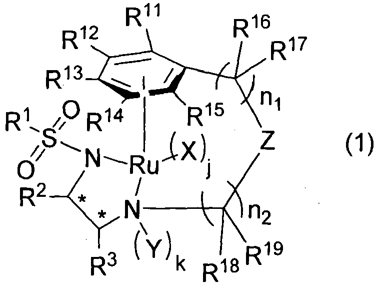 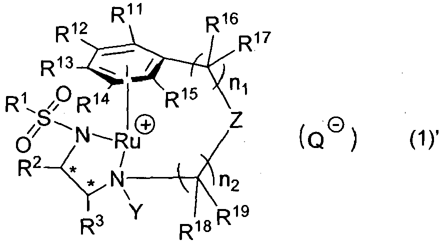 (where
R¹ represents an alkyl group having 1 to 10 carbon atoms; a halogenated alkyl group having 1 to 10 carbon atoms; a 10-camphoryl group; an amino group which may be substituted with one or two alkyl groups having 1 to 10 carbon atoms; or an aryl group (provided that the aryl group may be substituted with one or more selected from alkyl groups having 1 to 10 carbon atoms, halogenated alkyl groups having 1 to 10 carbon atoms, halogen atoms, cyano groups (-CN), amino groups, alkylamino groups (-NR²⁰R²¹), 5- or 6-membered cyclic amino groups, acylamino groups (-NH-CO-R²⁰), hydroxyl groups, alkoxy groups (-OR²⁰), acyl groups (-CO-R²⁰), carboxyl groups, alkoxycarbonyl groups (-COOR²⁰), phenoxycarbonyl groups, mercapto groups, alkylthio groups (-SR²⁰), silyl groups (-SiR²⁰R²¹R²²), and nitro groups (-NO₂)),
R²⁰, R²¹, and R²² each independently represent a hydrogen atom, an alkyl group having 1 to 10 carbon atoms, or a cycloalkyl group having 3 to 10 carbon atoms,
Y represents a hydrogen atom,
X represents a trifluoromethanesulfonyloxy group, a p-toluenesulfonyloxy group, a methanesulfonyloxy group, a benzenesulfonyloxy group, a hydrogen atom, or a halogen atom,
Q_{Θ} represents a counter anion,
j and k each represent 0 or 1, provided that cases where j+k=1 are excluded,
R² and R³ each independently represent a hydrogen atom; an alkyl group having 1 to 10 carbon atoms; a phenyl group (provided that the phenyl group may be substituted with one or more selected from alkyl groups having 1 to 10 carbon atoms, alkoxy groups having 1 to 10 carbon atoms, and halogen atoms); or a cycloalkyl group having 3 to 8 carbon atoms, or R² and R³ may together form a ring,
R¹¹, R¹², R¹³, R¹⁴, and R¹⁵ each independently represent a hydrogen atom, an alkyl group having 1 to 10 carbon atoms, or an alkoxy group having 1 to 10 carbon atoms,
R¹⁶, R¹⁷, R¹⁸, and R¹⁹ each independently represent a hydrogen atom, a hydroxyl group, an alkyl group having 1 to 10 carbon atoms, or an alkoxy group having 1 to 10 carbon atoms, or R¹⁶, R¹⁷, and the carbon atom to which R¹⁶ and R¹⁷ are bonded and/or R¹⁸, R¹⁹, and the carbon atom to which R¹⁸ and R¹⁹ are bonded may form a carbonyl group(s),
Z represents an oxygen atom, a sulfur atom, or a methylene,
n₁ represents 1 or 2, and n₂ represents any integer of 1 to 3, and
each * indicates an asymmetric carbon atom (provided that when R² and/or R³ is/are a hydrogen atom(s), the carbon atom to which the hydrogen atom is bonded is not an asymmetric carbon atom)),
the β-keto-α-aminocarboxylic acid ester is represented by the following general formula (2): 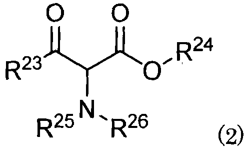 (where
R²³ represents a hydrocarbon group which has 11 to 21 carbon atoms and which may be substituted with one or more hydroxyl groups,
R²⁴ represents a hydrogen atom or a hydrocarbon group having 1 to 10 carbon atoms, and
R²⁵ and R²⁶, which may be the same or different, each represent a hydrogen atom, an alkyl group which has 1 to 10 carbon atoms and which may be substituted with one or more selected from halogen atoms and hydroxyl groups, an acyl group which has 1 to 24 carbon atoms and which may be substituted with one or more selected from halogen atoms and hydroxyl groups, or an amino-protecting group, or R²⁵ and R²⁶ may form, together with the adjacent nitrogen atom, a heterocycle which may be substituted with one or more hydroxyl groups), and
the optically active β-hydroxy-α-aminocarboxylic acid ester is represented by the following general formula (3) or (4): 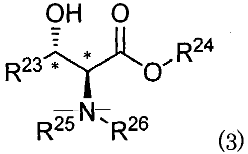 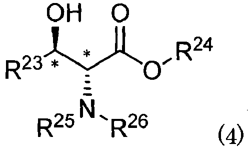 (where
each * indicates an asymmetric carbon atom, and
R²³ R²⁴ R²⁵ and R²⁶ are the same as those described above).

Hereinafter, the ruthenium complex represented by general formula (1) and a method for synthesizing the ruthenium complex are described first. Subsequently, a description is given of the ß-keto-α-aminocarboxylic acid ester represented by general formula (2) and the ß-hydroxy-α-aminocarboxylic acid esters represented by general formulae (3) and (4). Then, a description is given of a process for producing the β-hydroxy-α-aminocarboxylic acid esters represented by general formulae (3) and (4) by carrying out an asymmetric reduction reaction of the ß-keto-α-aminocarboxylic acid ester represented by general formula (2) by use of the ruthenium complex represented by general formula (1) as a catalyst.

### <Ruthenium Complex>

The ruthenium complex represented by general formula (1) is such that an aromatic compound (arene) moiety is coordinated to a ruthenium atom, and that a hetero atom such as an oxygen atom or a sulfur atom is introduced to a chain moiety linking the aromatic compound (arene) moiety and a diamine moiety, or the aromatic compound (arene) moiety and the diamine moiety are linked through a carbon chain.

In addition, the ruthenium complex represented by general formula (1) has a tridentate ligand whose two nitrogen atoms in the diamine ligand are bonded to the ruthenium atom by covalent bonding or coordination bonding, and whose aromatic compound (arene) moiety linked to the diamine is also coordinated to the ruthenium atom. In addition, in the ruthenium complex, a hetero atom such as an oxygen atom or a sulfur atom is introduced to the chain moiety linking the aromatic compound (arene) moiety and the diamine moiety.

Each sign * in general formula (1) indicates that the carbon atom to which the sign * is attached may be an asymmetric carbon atom. When the carbon atom is an asymmetric carbon atom, the ruthenium complex may be an optically active compound, a mixture of optically active compounds, or a racemic mixture (including a racemic compound), in terms of the asymmetric carbon atom. In a preferred mode of the present invention, the ruthenium complex is an optically active compound, when any of these carbon atoms is an asymmetric carbon atom.

However, as described later, when R² and/or R³ is/are a hydrogen atom(s), the carbon atom to which the hydrogen atom is bonded is not an asymmetric carbon atom.

In general formula (1) of the present invention, R¹ represents
an alkyl group having 1 to 10 carbon atoms;
a halogenated alkyl group having 1 to 10 carbon atoms;
a 10-camphoryl group;
an amino group which may be substituted with one or two alkyl groups having 1 to 10 carbon atoms; or
an aryl group (provided that the aryl group may be substituted with one or more selected from alkyl groups having 1 to 10 carbon atoms, halogenated alkyl groups having 1 to 10 carbon atoms, halogen atoms, cyano groups (-CN), amino groups, alkylamino groups (-NR²⁰R²¹), 5- or 6-membered cyclic amino groups, acylamino groups (-NH-CO-R²⁰), hydroxyl groups, alkoxy groups (-OR²⁰), acyl groups (-CO-R²⁰), carboxyl groups, alkoxycarbonyl groups (-COOR²⁰), phenoxycarbonyl groups, mercapto groups, alkylthio groups (-SR²⁰), silyl groups (-SiR²⁰R²¹R²²), and nitro groups (-NO₂)).

Examples of the alkyl group having 1 to 10 carbon atoms represented by R¹ in general formula (1) of the present invention include linear or branched alkyl groups having 1 to 10 carbon atoms, and preferably 1 to 5 carbon atoms. Specific examples of the alkyl groups include a methyl group, an ethyl group, a n-propyl group, an isopropyl group, a n-butyl group, an isobutyl group, a s-butyl group, a t-butyl group, a n-pentyl group, a n-hexyl group, a n-heptyl group, a n-octyl group, a n-nonyl group, a n-decyl group, and the like.

Examples of the halogenated alkyl group having 1 to 10 carbon atoms represented by R¹ in general formula (1) of the present invention include alkyl groups having 1 to 10 carbon atoms which are the same as the above-described linear or branched alkyl groups such as a methyl group, an ethyl group, a n-propyl group, an isopropyl group, a n-butyl group, and a n-hexyl group, except that the alkyl groups are substituted with one or more halogen atoms such as fluorine atoms, chlorine atoms, and bromine atoms. Examples of the halogenated alkyl groups include perfluoroalkyl groups such as a trifluoromethyl group, a pentafluoroethyl group, and a heptafluoropropyl group.

Examples of the alkyl group which has 1 to 10 carbon atoms and which may be included in the amino group represented by R¹ in general formula (1) of the present invention include linear or branched alkyl groups having 1 to 10 carbon atoms, and preferably 1 to 5 carbon atoms. Specific examples of the alkyl group include a methyl group, an ethyl group, a n-propyl group, an isopropyl group, a n-butyl group, an isobutyl group, a s-butyl group, a t-butyl group, a n-pentyl group, a n-hexyl group, a n-heptyl group, a n-octyl group, a n-nonyl group, and a n-decyl group.

Examples of the aryl group in general formula (1) of the present invention (provided that the aryl group may be substituted with one or more selected from alkyl groups having 1 to 10 carbon atoms, halogenated alkyl groups having 1 to 10 carbon atoms, halogen atoms, cyano groups (-CN), amino groups, alkylamino groups (-NR²⁰R²¹), 5- or 6-membered cyclic amino groups, acylamino groups (-NH-CO-R²⁰), hydroxyl groups, alkoxy groups (-OR²⁰), acyl groups (-CO-R²⁰), carboxyl groups, alkoxycarbonyl groups (-COOR²⁰), phenoxycarbonyl groups, mercapto groups, alkylthio groups (-SR²⁰), silyl groups (-SiR²⁰R²¹R²²), and nitro groups (-NO₂)) include monocyclic, polycyclic, or condensed cyclic aryl groups having 1 to 20 carbon atoms, and preferably 6 to 12 carbon atoms, such as a phenyl group and a naphthyl group.

Examples of the aryl groups include a phenyl group, o-, m-, and p-tolyl groups, o-, m-, and p-ethylphenyl groups, o-, m-, and p-isopropylphenyl groups, o-,m-, and p-t-butylphenyl groups, a 2,4,6-trimethylphenyl group, a 3,5-xylyl group, a 2,4,6-triisopropylphenyl group, o-, m-, and p-trifluoromethylphenyl groups, o-, m-, and p-fluorophenyl groups, o-, m-, and p-chlorophenyl groups, a pentafluorophenyl group, and the like.

Note that R²⁰, R²¹, and R²² each independently represent a hydrogen atom, an alkyl group having 1 to 10 carbon atoms, or a cycloalkyl group having 3 to 10 carbon atoms. R²⁰, R²¹, and R²² are described later.

Hereinafter, a further description is given of the alkyl groups having 1 to 10 carbon atoms, the halogenated alkyl groups having 1 to 10 carbon atoms, the halogen atoms, the alkylamino groups (-NR²⁰R²¹), the 5- or 6-membered cyclic amino groups, the acylamino groups (-NH-CO-R²⁰), the alkoxy groups (-OR²⁰), the acyl groups (-COR²⁰), the alkoxycarbonyl groups (-COOR²⁰), the alkylthio groups (-SR²⁰), and the silyl groups (-SiR²⁰R²¹R²²), which may be selected as the substituent in the aryl group.

Examples of the alkyl group having 1 to 10 carbon atoms which may be selected as the substituent in the aryl group include the alkyl groups described above. Examples of the halogenated alkyl group having 1 to 10 carbon atoms which may be selected as the substituent in the aryl group include the halogenated alkyl groups described above, and an example of which is a perfluoroalkyl group.

Examples of the halogen atom which may be selected as the substituent in the aryl group include a fluorine atom, a chlorine atom, and the like.

Examples of the alkylamino group which may be selected as the substituent in the aryl group and which is represented by -NR²⁰R²¹ (where R²⁰ and R²¹ each independently represent a hydrogen atom, an alkyl group having 1 to 10 carbon atoms, or a cycloalkyl group having 3 to 10 carbon atoms) include monoalkylamino groups and dialkylamino groups such as an N-methylamino group, an N,N-dimethylamino group, an N,N-diisopropylamino group, and an N-cyclohexylamino group.

Examples of the 5- or 6-membered cyclic amino group which may be selected as the substituent in the aryl group include 5- or 6- membered unsaturated or saturated heterocyclic groups having one or two nitrogen atoms such as a pyrrolidinyl group, a piperidino group, and a morpholyl group.

Examples of the acylamino group which may be selected as the substituent in the aryl group and which is represented by -NH-CO-R²⁰ (where R²⁰ represents a hydrogen atom, an alkyl group having 1 to 10 carbon atoms, or a cycloalkyl group having 3 to 10 carbon atoms) include a formylamino group, an acetylamino group, a propionylamino group, a pivaloylamino group, a pentanoylamino group, a hexanoylamino group, and the like.

Examples of the alkoxy group which may be selected as the substituent in the aryl group and which is represented by -OR²⁰ (where R²⁰ represents a hydrogen atom, an alkyl group having 1 to 10 carbon atoms, or a cycloalkyl group having 3 to 10 carbon atoms) include a methoxy group, an ethoxy group, a n-propoxy group, an isopropoxy group, a n-butoxy group, a s-butoxy group, an isobutoxy group, a t-butoxy group, a n-pentyloxy group, a 2-methylbutoxy group, a 3-methylbutoxy group, a 2,2-dimethylpropyloxy group, a n-hexyloxy group, a 2-methylpentyloxy group, a 3-methylpentyloxy group, a 4-methylpentyloxy group, a 5-methylpentyloxy group, a cyclohexyloxy group, and the like.

Examples of the acyl group; which may be selected as the substituent in the aryl group and which is represented by -CO-R²⁰ (where R²⁰ represents a hydrogen atom, an alkyl group having 1 to 10 carbon atoms, or a cycloalkyl group having 3 to 10 carbon atoms) include a formyl group, an acetyl group, a propionyl group, a butyryl group, a pivaloyl group, a pentanoyl group, a hexanoyl group, and the like.

Examples of the alkoxycarbonyl group which may be selected as the substituent in the aryl group and which is represented by -COOR²⁰ (where R²⁰ represents a hydrogen atom, an alkyl group having 1 to 10 carbon atoms, or a cycloalkyl group having 3 to 10 carbon atoms) include a methoxycarbonyl group, an ethoxycarbonyl group, a n-propoxycarbonyl group, an isopropoxycarbonyl group, a n-butoxycarbonyl group, a t-butoxycarbonyl group, a pentyloxycarbonyl group, a hexyloxycarbonyl group, a 2-ethylhexyloxycarbonyl group, and the like.

Examples of the alkylthio group which may be selected as the substituent in the aryl group and which is represented by -SR²⁰ (where R²⁰ represents a hydrogen atom, an alkyl group having 1 to 10 carbon atoms, or a cycloalkyl group having 3 to 10 carbon atoms) include a methylthio group, an ethylthio group, a n-propylthio group, an isopropylthio group, a n-butylthio group, a s-butylthio group, an isobutylthio group, a t-butylthio group, a pentylthio group, a hexylthio group, a cyclohexylthio group, and the like.

Examples of the silyl group which may be selected as the substituent in the aryl group and which is represented by -SiR²⁰R²¹R²² (where R²⁰, ^{R21}, and R²² each independently represent a hydrogen atom, an alkyl group having 1 to 10 carbon atoms, or a cycloalkyl group having 3 to 10 carbon atoms) include a trimethylsilyl group, a triisopropylsilyl group, a t-butyldimethylsilyl group, a t-butyldiphenylsilyl group, a triphenylsilyl group, and the like.

Here, examples of the alkyl group having 1 to 10 carbon atoms in each of the above-described definitions of R²⁰, R²¹, and R²² include the alkyl groups described above. Examples of the cycloalkyl group having 3 to 10 carbon atoms in each of the above-described definitions of R²⁰, R²¹, and R²² include monocyclic, polycyclic, or condensed cyclic, saturated or unsaturated, 3- to 7-membered cycloalkyl groups having 3 to 10 carbon atoms.

In general formula (1) of the present invention, Y represents a hydrogen atom, and X represents a trifluoromethanesulfonyloxy group, a p-toluenesulfonyloxy group, a methanesulfonyloxy group, a benzenesulfonyloxy group, a hydrogen atom, or a halogen atom, and preferably a halogen atom. A specific preferred example of X is a chlorine atom.

Q_{Θ} in the formula (1)' represents a counter anion. Specific examples of the counter anion include borate ions such as a tetrafluoroborate ion (BF₄⁻), a tetraphenylborate ion (B(C₆F₅)₄⁻), and a BAr_{F} ion (B(3,5-(CF₃)₂C₆F₃)₄⁻); and ions such as SbF₆⁻, CF₃COO⁻, CH₃COO⁻, PF6⁻, NO₃⁻, ClO₄⁻, SCN⁻, OCN⁻, ReO₄⁻ and MoO₄⁻

The hydrogen atom represented by each of Y and X in general formula (1) may be not only an ordinary hydrogen atom, but also an isotope atom of hydrogen. A preferred isotope atom is a deuterium atom.

In general formula (1), k and j each represent an integer of 0 or 1, provided that cases where j+k=1 are excluded. In other words, when k is 1, j is also 1, whereas when k is 0, j is also 0.

Meanwhile, R² and R³ in general formula (1) of the present invention each independently represent
a hydrogen atom;
an alkyl group having 1 to 10 carbon atoms;
a phenyl group (provided that the phenyl group may be substituted with one or more selected from alkyl groups having 1 to 10 carbon atoms, alkoxy groups having 1 to 10 carbon atoms, and halogen atoms); or
a cycloalkyl group having 3 to 8 carbon atoms.

Alternatively, R² and R³ may together form a ring.

Examples of the alkyl group having 1 to 10 carbon atoms represented by each of R² and R³ in general formula (1) of the present invention include linear or branched alkyl groups having 1 to 10 carbon atoms, and preferably 1 to 5 carbon atoms. Specific examples of the alkyl groups include a methyl group, an ethyl group, a n-propyl group, an isopropyl group, a n-butyl group, an isobutyl group, a s-butyl group, a t-butyl group, a n-pentyl group, a n-hexyl group, a n-heptyl group, a n-octyl group, a n-nonyl group, a n-decyl group, and the like.

The phenyl group represented by each of R² and R³ in general formula (1) of the present invention may be substituted with one or more selected from alkyl groups having 1 to 10 carbon atoms, alkoxy groups having 1 to 10 carbon atoms, and halogen atoms. Examples of the alkyl groups having 1 to 10 carbon atoms include the alkyl groups described above. Examples of the alkoxy groups having 1 to 10 carbon atoms include linear or branched alkoxy groups having 1 to 10 carbon atoms, and preferably 1 to 5 carbon atoms. Specific examples of the alkoxy groups include a methoxy group, an ethoxy group, a n-propoxy group, an isopropoxy group, a n-butoxy group, an isobutoxy group, a s-butoxy group, a t-butoxy group, a n-pentyloxy group, a n-hexyloxy group, a n-heptyloxy group, a n-octyloxy group, a n-nonyloxy group, a n-decyloxy group, and the like. Examples of the halogen atom include a fluorine atom, a chlorine atom, and a bromine atom.

Examples of the cycloalkyl group having 3 to 8 carbon atoms represented by each of R² and R³ in general formula (1) of the present invention include monocyclic, polycyclic, or cross-linked cycloalkyl groups having 3 to 8 carbon atoms, and preferably 5 to 8 carbon atoms. Specific examples of the cycloalkyl groups include a cyclopropyl group, a cyclobutyl group, a cyclopentyl group, a cyclohexyl group, a cycloheptyl group, a cyclooctyl group, and the like. These cycloalkyl groups may be substituted with alkyl groups or the like such as methyl groups, isopropyl groups, and t-butyl groups.

In addition, when R² and R³ in general formula (1) of the present invention together form a ring, R² and R³ together represent a linear or branched alkylene group having 2 to 10 carbon atoms, and preferably 3 to 10 carbon atoms, and the alkylene group, together with the adjacent carbon atoms, forms a 4- to 8-membered, preferably 5- to 8-membered cycloalkane ring. Preferred examples of the cycloalkane ring include a cyclopentane ring, a cyclohexane ring, and a cycloheptane ring. These rings may have alkyl groups and the like, such as methyl groups, isopropyl groups, and t-butyl groups, as substituents.

In the present invention, R¹¹, R¹², R¹³, R¹⁴, and R¹⁵ in the arene moiety represented by general formula (1) each independently represent
a hydrogen atom;
an alkyl group having 1 to 10 carbon atoms; or
an alkoxy group having 1 to 10 carbon atoms.

Examples of the alkyl group having 1 to 10 carbon atoms include the alkyl groups described above. Specific examples of the alkyl groups include a methyl group, an ethyl group, a n-propyl group, an isopropyl group, a n-butyl group, an isobutyl group, a s-butyl group, a t-butyl group, a n-pentyl group, a n-hexyl group, a n-heptyl group, a n-octyl group, a n-nonyl group, a n-decyl group, and the like.

Examples of the alkoxy group having 1 to 10 carbon atoms include the linear or branched alkoxy groups described above. Specific examples of the alkoxy groups include a methoxy group, an ethoxy group, a n-propoxy group, an isopropoxy group, a n-butoxy group, an isobutoxy group, a s-butoxy group, a t-butoxy group, a n-pentyloxy group, a n-hexyloxy group, a n-heptyloxy group, a n-octyloxy group, a n-nonyloxy group, a n-decyloxy group, and the like.

R¹⁶, R¹⁷, R¹⁸, and R¹⁹ which represent substituents introduced to the carbon atoms in the chain moiety linking the arene moiety and the diamine moiety in general formula (1) each independently represent
a hydrogen atom;
a hydroxyl group;
an alkyl group having 1 to 10 carbon atoms; or
an alkoxy group having 1 to 10 carbon atoms.

Examples of the alkyl group having 1 to 10 carbon atoms include the alkyl groups described above. Specific examples of the alkyl groups include a methyl group, an ethyl group, a n-propyl group, an isopropyl group, a n-butyl group, an isobutyl group, a s-butyl group, a t-butyl group, a n-pentyl group, a n-hexyl group, a n-heptyl group, a n-octyl group, a n-nonyl group, a n-decyl group, and the like.

Examples of the alkoxy group having 1 to 10 carbon atoms include the linear or branched alkoxy groups described above. Specific examples of the alkoxy groups include a methoxy group, an ethoxy group, a n-propoxy group, an isopropoxy group, a n-butoxy group, an isobutoxy group, a s-butoxy group, a t-butoxy group, a n-pentyloxy group, a n-hexyloxy group, a n-heptyloxy group, a n-octyloxy group, a n-nonyloxy group, a n-decyloxy group, and the like.

Preferred examples of the -(-C(R¹⁶)R¹⁷-)n₁- group include a -CH₂- group, a -CH(CH₃)- group, a -CO- group, and the like, but the -(-C(R¹⁶)R¹⁷-)n₁- group is not limited to these groups.

Preferred examples of the -(-C(R¹⁸)R¹⁹-)n₂- group include a -CH₂- group, a -CH(CH₃)- group, a -CO- group, and the like, but the -(-C(R¹⁸)R¹⁹-)n₂- group is not limited to these groups.

Z in general formula (1) is an oxygen atom (-O-), a sulfur atom (-S-), or a methylene (-CH₂-).

In addition, m represents 1 or 2, and preferably 1, and n₂ represents any integer of 1 to 3, and preferably 2.

### <Method for Synthesizing Ruthenium Complex>

The above-described ruthenium complex can be synthesized, for example, by the method shown in the following scheme 1:

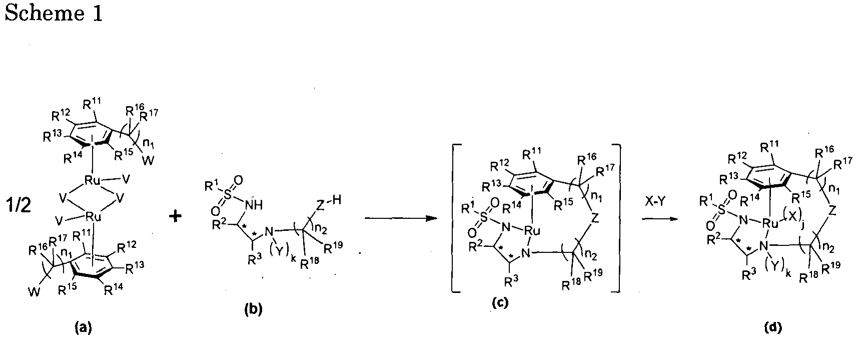

In scheme 1, R¹, R², R³, R¹¹ to R¹⁵, and R¹⁶ to R¹⁹ represent the substituents as described above, Y represents a hydrogen atom or a deuterium atom, and Z represents an oxygen atom, a sulfur atom, or a methylene. In a ruthenium arene dimer (a), W represents a halogen atom, an alkanesulfonyloxy group which may be substituted with one or more halogen atoms, or an arenesulfonyloxy group which may be substituted with an alkane(s), and V represents a halogen atom. n₁ represents 1 or 2, and n₂ represent any integer of 1 to 3.

As shown in scheme 1, a thioether formation or ether formation reaction is conducted simultaneously with the complexation by reacting, in the presence of an appropriate base, the ruthenium arene dimer (a) having halogen atoms or the like in terminals of substituents on the arenes with a diamine (b) having a hydroxyl group or a thiol group in a terminal of a chain moiety bonded to the nitrogen atom to which the sulfonyl group is not bonded. Thus, a ruthenium-diamine complex (d), which is the target complex, can be synthesized directly or through an amide complex (c). When the synthesis is conducted through the amide complex (c), the amide complex (c) can be converted to the diamine complex (d) by adding an appropriate acid to the amide complex (c).

Examples of the halogen atom, the alkanesulfonyloxy group which may be substituted with one or more halogen atoms, and the arenesulfonyloxy group which may be substituted with an alkane(s), which are represented by W in the ruthenium arene dimer (a), include a chlorine atom, a bromine atom, an iodine atom, a methanesulfonyloxy group, a p-toluenesulfonyloxy group, a benzenesulfonyloxy group, a trifluoromethanesulfonyloxy group, and the like. In addition, the halogen atoms represented by Vs each represent any one of a chlorine atom, a bromine atom, and an iodine atom, and all of the Vs may be the same halogen atoms, or the Vs may be a combination of different halogen atoms.

In the diamine (b), Z is an oxygen atom, a sulfur atom, or a methylene, and Y represents a hydrogen atom.

Examples of a base used for synthesizing the amide complex (c) include inorganic bases such as LiOH, NaOH, KOH, K₂CO₃, and Cs₂CO₃; and metal alkoxides such as sodium methoxide and potassium methoxide. The amount of the base added is 2 mol or more relative to 1 mol of ruthenium atoms. A solvent used in this case is not particularly limited, and preferred examples thereof include ethers such as diethyl ether and tetrahydrofuran; aromatic hydrocarbons such as toluene and xylene; halogen-containing hydrocarbon solvents such as dichloromethane and 1,2-dichloroethane; aprotic polar solvents such as acetonitrile and N,N-dimethylformamide; and the like, and particularly preferred examples thereof include dichloromethane and toluene. In addition, water may be used as another solvent, and this reaction can be carried out as a reaction of a two-layer system of water with an organic solvent. In this case, it is preferable to carry out the reaction by using a phase transfer catalyst. Examples of the phase transfer catalyst used here include tetrabutylammonium chloride, tetrabutylammonium bromide, tetrabutylammonium iodide, tetraethylammonium chloride, tetraethylammonium bromide, tetraethylammonium iodide, triethylbenzylammonium chloride, triethylbenzylammonium bromide, triethylbenzylammonium iodide, and the like.

Examples of the acid (X-Y) used for the conversion from the amide complex (c) to the diamine complex (d) include hydrochloric acid, hydrobromic acid, hydroiodic acid, and the like.

The solvent in which this reaction is carried out is not particularly limited. After the synthesis of the amide complex (c), the conversion from the amide complex (c) to the diamine complex (d) can be carried out, without isolation of the amide complex (c), by performing a reaction directly in the same system in the presence of the same solvent. Alternatively, the conversion to the diamine complex (d) may be carried out by isolating the amide complex (c), and then performing a reaction using an appropriate different solvent.

Preferred examples of the base used for the direct synthesis of the diamine complex (d) include tertiary organic amines such as trimethylamine, triethylamine, triisopropylamine, and diisopropylethylamine, and particularly preferred examples thereof include triethylamine and diisopropylethylamine. In this case, the amount of the base added is equimolar or more relative to the ruthenium atoms.

The solvent used in this case is not particularly limited. Preferred examples of the solvent include ethers such as diethyl ether and tetrahydrofuran; alcohols such as methanol, ethanol, and isopropanol aromatic hydrocarbons such as toluene and xylene; halogen-containing solvents such as dichloromethane and 1,2-dichloroethane; aprotic polar solvents such as acetonitrile and N,N-dimethylformamide; and the like, and particularly preferred examples thereof include dichloromethane and isopropanol.

Alternatively, in another method for synthesizing the complex of the present invention, a ruthenium arene dimer (e) having hydroxyl groups or thiol groups in terminals of substituents of the arenes and a diamine (f) having a halogen atom or the like in a terminal of a chain moiety bonded to the nitrogen atom to which a sulfonyl group is not bonded can also be used as raw materials, as shown in the following scheme 2. 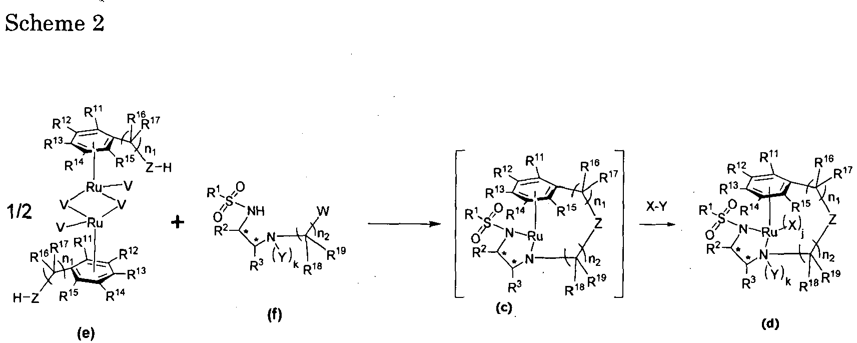 (in scheme 2, each sign has the same meaning as that in scheme 1).

In this scheme 2, the combination of positions of the hydroxyl group or thiol group and the leaving group such as a halogen atom is inversed as compared with that in scheme 1. Also in this case, a thioether formation or ether formation reaction is conducted simultaneously with the complexation by reacting these substances in the presence of an appropriate base. Thus, the ruthenium-diamine complex (d) or a cationic diamine complex (g), which are the target complexes, can be similarly synthesized directly or through the amide complex (c). When the synthesis is conducted through the amide complex (c), the amide complex (c) can be converted to the complex (d) or (g) by adding an appropriate acid to the amide complex (c). The base, solvent, and the like used in this reaction are the same as those described above.

Moreover, the complex of the present invention can also be produced by a method as shown in the following scheme 3. 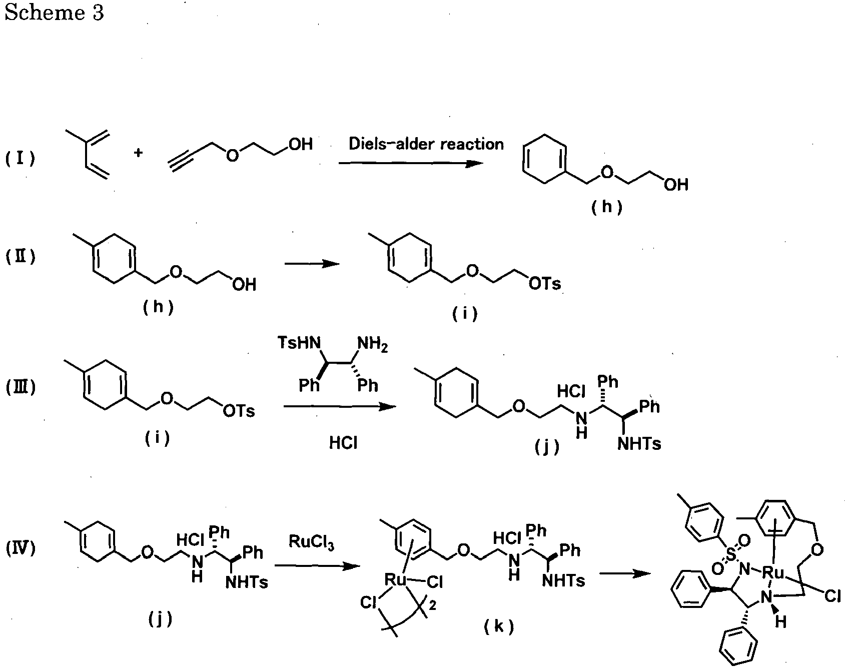
(I) A compound (h) having a 1,4-cyclohexadiene skeleton is synthesized by a Diels-Alder reaction.
(II) A compound (i) having a leaving group in its terminal is synthesized by tosylation or the like of the compound (h) obtained in (I).
(III) A diamine (j) having a cyclohexadiene skeleton is synthesized by a reaction of the compound (i) with TsDPEN (N-(p-toluenesulfonyl)-1,2-diphenylethylenediamine).
(IV) The target monomer complex can be obtained by a reaction of the obtained diamine (j) with ruthenium trichloride through a ruthenium dimer (k).

By this method, the ruthenium complex represented by general formula (1) can be produced.

The use of such a ruthenium complex as a catalyst makes it possible to produce an optically active β-hydroxy-α-aminocarboxylic acid ester in a high yield at a high catalytic efficiency with a high selectivity

Note that the preparation of the ruthenium complex of the present invention is generally carried out at 120°C or below, and preferably 100°C or below.

In addition, as for an asymmetric reduction reaction described below, the reaction may be carried out by using the isolated amide complex (c) or diamine complex (d) as a catalyst, or the reaction may be carried out by using the reaction liquid of the complex preparation as it is without isolation of the complex (an in situ method).

### <β-Keto-α-aminocarboxylic Acid Ester>

In the β-keto-α-aminocarboxylic acid ester represented by general formula (2), R²³ represents a hydrocarbon group which may be substituted with one or more hydroxyl groups and which has 11 to 21 carbon atoms, preferably 11 to 18 carbon atoms, and further preferably 11 to 15 carbon atoms. The hydrocarbon group (which may be substituted with one or more hydroxyl groups) may be acyclic or cyclic. When the hydrocarbon group is acyclic, the hydrocarbon group may be a linear or branched saturated hydrocarbon group or a linear or branched unsaturated hydrocarbon group, and is preferably a linear saturated hydrocarbon group. When the hydrocarbon group is cyclic, the hydrocarbon group may likewise be a saturated cyclic hydrocarbon group or an unsaturated cyclic hydrocarbon group, and is preferably a saturated cyclic hydrocarbon group. R²³ is preferably a linear saturated fatty acid group having 11 to 15 carbon atoms, for example. Specifically, R²³ is preferably a pentadecyl group, a 1-hydroxypentadecyl group, or a dodecyl group. R²³ is particularly preferably a pentadecyl group or a 1-hydroxypentadecyl group, from the viewpoint of usefulness of the compound (2).

In the β-keto-α-aminocarboxylic acid ester represented by general formula (2), R²⁴ represents a hydrogen atom or a hydrocarbon group having 1 to 10 carbon atoms, preferably a hydrogen atom, or a hydrocarbon group having 1 to 5 carbon atoms, and further preferably a hydrocarbon group having 1 to 4 carbon atoms. The hydrocarbon group may be acyclic or cyclic. When the hydrocarbon group is acyclic, the hydrocarbon group may be a linear or branched saturated hydrocarbon group or a linear or branched unsaturated hydrocarbon group, and is preferably a linear saturated hydrocarbon group. When the hydrocarbon group is cyclic, the hydrocarbon group may likewise be a saturated cyclic hydrocarbon group or an unsaturated cyclic hydrocarbon group, and is preferably a saturated cyclic hydrocarbon group. R²⁴ is preferably a saturated hydrocarbon group having 1 to 4 carbon atoms. Specifically, R²⁴ is preferably a methyl group, an ethyl group, a propyl group, an isopropyl group, an isobutyl group, or a tert-butyl group. From the viewpoint of the easiness of synthesis of the raw material, R²⁴ is further preferably a methyl group, an ethyl group, or a propyl group, and particularly preferably a methyl group.

In the β-keto-α-aminocarboxylic acid ester represented by general formula (2), R²⁵ and R²⁶ may be the same or different, and are preferably different from each other. R²⁵ and R²⁶ each represent a hydrogen atom, an alkyl group which has 1 to 10 carbon atoms and which may be substituted with one or more selected from halogen atoms and hydroxyl groups, an acyl group which has 1 to 24 carbon atoms and which may be substituted with one or more selected from halogen atoms and hydroxyl groups, or an amino-protecting group.

When any of R²⁵ and R²⁶ is an alkyl group having 1 to 10 carbon atoms (which may be substituted with one or more halogen atoms or one or more hydroxyl groups), an alkyl group having 1 to 5 carbon atoms, and particularly preferably 1 to 3 carbon atoms is preferably selected as the alkyl group. In addition, the alkyl group may be linear or branched, and is preferably linear. Specific examples of the alkyl group include those described as the specific examples of the alkyl group having 1 to 10 carbon atoms described above.

When any of R²⁵ and R²⁶ is an acyl group having 1 to 24 carbon atoms (which may be substituted with one or more halogen atoms or one or more hydroxyl groups), an acyl group having preferably 1 to 21 carbon atoms, and particularly preferably 1 to 18 carbon atoms is selected as the acyl group. In addition, the acyl group may be a saturated acyl group or an unsaturated acyl group, and is preferably a saturated acyl group. Specific preferred examples of the acyl group include a formyl group, an acetyl group, a trifluoroacetyl group, a trichloroacetyl group, a monochloroacetyl group, a benzoyl group, an octadecanoyl group, a 2-hydroxyoctadecanoyl group, and a 2-oxooctadecanoyl group.

When any of R²⁵ and R²⁶ is an amino-protecting group, a group described in Protective Groups in Organic Synthesis 3rd ed. (Theodora W. Greene and Peter G. M. Wuts Ed., Wiley-Interscience: New York, 1999) can be selected as the amino-protecting group, for example. Specific preferred examples of the amino-protecting group include alkoxycarbonyl groups such as a methoxycarbonyl group, an ethoxycarbonyl group, a benzyloxycarbonyl group, and a tert-butoxycarbonyl group, and sulfonyl groups such as a p-nitrobenzenesulfonyl group.

In the β-keto-α-aminocarboxylic acid ester represented by general formula (2), R²⁵ and R²⁶ may form, together with the adjacent nitrogen atom, a heterocycle which may be substituted with one or more hydroxyl groups. When the heterocyclic is formed, for example, a phthaloyl group is selected as R²⁵ and R²⁶, and a phthalimide group is formed together with the adjacent nitrogen atom.

As for R²⁵ and R²⁶, it is preferable that one of R²⁵ and R²⁶ be an acyl group which has 1 to 24 carbon atoms and which may have a substituent(s), and the other be a hydrogen, or that R²⁵ and R²⁶, together with the adjacent nitrogen atom, form a heterocycle.

More preferably, one of R²⁵ and R²⁶ is an acyl group which has 1 to 24 carbon atoms and which may be substituted with one or more selected from halogen atoms and hydroxyl groups, and the other is a hydrogen. In this case, the acyl group which has 1 to 24 carbon atoms and which may be substituted with one or more selected from halogen atoms and hydroxyl groups is preferably a formyl group, an acetyl group, a benzoyl group, or an octadecanoyl group, from the viewpoint of easiness of deprotection.

As for a method for producing the compound (2), the compound (2) can be produced by known methods such as a method in which a β-keto ester is treated with sodium nitrite, followed by oximation at the α position, and then an amino group is formed by reduction of only the oxime.

### <Optically Active β-Hydroxy-α-aminocarboxylic Acid Ester>

In the optically active β-hydroxy-α-aminocarboxylic acid ester represented by general formula (3) or (4), the definitions of R²³, R²⁴ R²⁵ and R²⁶ are the same as those described above.

In general formula (3) and (4), each * indicates an asymmetric carbon atom. Since the compound (3) or (4) has two asymmetric carbon atoms, two diastereomers are present. A compound having a relative configuration as that of the compound (3) or (4) is referred to as an anti isomer. The other diastereomer is referred to as a syn isomer, and represented by the following general formula (5) or (6): 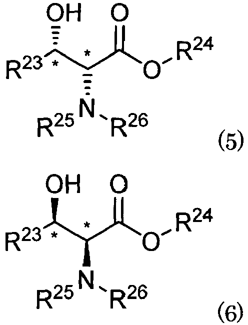 (where each * indicates an asymmetric carbon atom, and R²³, R²⁴, R²⁵, and R²⁶ are the same as those described above).

The present invention relates to a method for obtaining an optically active β-hydroxy-α-aminocarboxylic acid ester, in particular, an anti isomer of an optically active β-hydroxy-α-aminocarboxylic acid ester represented by general formula (3) or (4). It is particularly desirable to selectively obtain, of the anti isomers, the anti isomer of general formula (4), because the anti isomer of general formula (4) is an important intermediate of optically active ceramides useful as cosmetics components.

### <Method for Producing β-Hydroxy-α-aminocarboxylic Acid Ester>

The present invention relates to a method for producing a compound represented by general formula (3) or general formula (4), the method comprising performing an asymmetric reduction reaction of a compound represented by general formula (2) by use of a ruthenium complex represented by general formula (1) (hereinafter also simply referred to as a ruthenium complex) as a catalyst. Note that the present invention makes it possible to more selectively produce, of the anti isomers, the compound represented by general formula (4), because the compound represented by general formula (4) is an important intermediate of optically active ceramides having a naturally occurring configuration useful as cosmetics components. In the production method of the present invention, the compound represented by general formula (3) or general formula (4) can be obtained by an asymmetric reduction reaction which is conducted by preparing a reaction solution containing the ruthenium complex represented by general formula (1), the compound represented by general formula (2), and a hydrogen donor, and then allowing a reaction therebetween to proceed by, for example, heating this reaction solution or other procedures. Note that these materials may be added to the reaction solution in any order in the preparation of the reaction solution. In addition, in the production method, the reaction may be conducted by adding the hydrogen donor at once, or by adding the hydrogen donor continuously or intermittently. In addition, a common catalyst other than the ruthenium complex represented by general formula (1) may be contained as appropriate.

In addition, of the optically active β-hydroxy-α-aminocarboxylic acid esters represented by the general formula (3) and (4), the (2R,3R) isomer represented by general formula (4) is easily obtained as the major product in the production method of the present invention. Moreover, it is possible to achieve a formation ratio (2R,3R) isomer:(2S,3R) isomer of 85:15 to 100:0, more preferably 95:5 to 100:0. In other words, this production method makes it possible to obtain the anti isomer of an optically active β-hydroxy-α-aminocarboxylic acid ester with a high stereo selectivity.

Detailed conditions are described below.

The amount of the ruthenium complex used in the production method of the present invention is generally 1 to 1/100000 in terms of molar ratio relative to the β-keto-α-aminocarboxylic acid ester represented by general formula (2). However, the above-described reaction can be sufficiently and easily completed in the present invention, even when the molar ratio is 1/250 to 1/10000, or further 1/500 to 1/10000.

The hydrogen donor used in the production method of the present invention is not limited, as long as the hydrogen donor is capable of donating hydrogen to the β-keto-α-aminocarboxylic acid ester represented by general formula (2) during the reaction. Here, examples of usable hydrogen donors include metal hydrides such as borohydride compounds; and those generally used as hydrogen donors in hydrogen transfer reduction reaction, such as formic acid, salts thereof, and isopropanol. Specific examples of usable hydrogen donors include alcohols such as methanol, ethanol, n-propanol, and isopropanol; formic acid; sodium formate; ammonium formate; hydrogen; and the like. The amount of the hydrogen donor used may be any, as long as the amount is an equimolar amount or more to the catalyst in terms of hydride. Moreover, hydrogen gas can also be used as the hydrogen donor. The amount of hydrogen gas is preferably 1 to 100 equivalents, and particularly 1 to 10 equivalents to the β-keto-α-aminocarboxylic acid ester, in view of the reactivity In addition, because of easiness of handling, formic acid or hydrogen is preferably used, and formic acid is most preferably used.

The reaction pressure is not particularly limited, and the reaction is carried out at generally 0.05 to 0.2 MPa, and preferably normal pressure.

Meanwhile, when hydrogen gas is used as the hydrogen donor, the pressure is preferably 5 MPa or less, in general.

In the production method of the present invention, the reaction is preferably carried out in the coexistence of a base. Examples of the base which can be added for use in this reaction include inorganic bases such as sodium hydroxide, potassium hydroxide, sodium carbonate, and potassium carbonate; alkoxides such as sodium methoxide, sodium ethoxide, and sodium tert-butoxide; and bases such as ammonia and organic amines having 3 to 30 carbon atoms. Because of easiness of handling, it is preferable to add organic amines having 3 to 30 carbon atoms, and preferably 6 to 24 carbon atoms. Specific examples of the organic amines include triethylamine, tributylamines (for example, n-tributylamine), diisopropylethylamine, isopropyldimethylamine, trimethylamine, n-trioctylamine, iso-trioctylamine, 1,8-diazabicyclo[5.4.0]undec-7-ene, 1,5-diazabicyclo[4.3.0]non-5-ene, 1,4-diazabicyclo[2.2.2]octane, and the like. Preferred examples thereof include triethylamine, tributylamine, diisopropylethylamine, n-trioctylamine, 1,8-diazabicyclo[5.4.0]undec-7-ene, 1,5-diazabicyclo[4.3.0]non-5-ene, 2,6-lutidine, morpholine, 1-ethylpiperidine, 1-methylpiperidine, dicyclohexylmethylamine, dimethylaniline and diethylaniline and so on, and more preferred examples thereof include triethylamine, tributylamine diisopropylethylamine and dicyclohexylmethylamine. The amount of the coexistent base is not particularly limited, and is 1 to 100 equivalents, and preferably 1 to 10 equivalents to the β-keto-α-aminocarboxylic acid ester represented by formula (2). Moreover, a mixture of multiple bases may be used. In this case, the mixing ratio is not particularly limited.

When the hydrogen donor is formic acid, it is particularly preferable to use an organic amine as the base, among combinations of the above-described hydrogen donors and the above-described bases. In this case, formic acid and the amine are added to the reaction system separately, or an azeotrope of formic acid and an organic amine prepared in advance may be used. Preferred examples of the azeotrope of formic acid and an organic amine include an azeotrope of formic acid and triethylamine (5:2), and the like.

In this production method, the reaction temperature is -20°C to 180°C, preferably 0°C to 120°C, and further preferably 30°C to 100°C. A too-low reaction temperature leads to an uneconomical result, because the time to complete this reaction may be increased, or a large amount of the unreacted raw material may remain, in some cases. Meanwhile, a too-high reaction temperature is not preferable, because decomposition of the raw materials, the catalyst, or the like may occur in some cases. Accordingly, the reaction solution may be heated as appropriate in the production method of the present invention. By setting the temperature of the reaction solution to 0°C to 120°C, the asymmetric reduction reaction can be completed in 30 minutes to 72 hours. In particular, by setting the temperature of the reaction solution to 30 to 100°C, the asymmetric reduction reaction can be completed in 2 hours to 48 hours. The heating temperature and the reaction time can be adjusted depending on the optically active compound to be obtained. More particularly, by appropriately setting reaction conditions, the reaction time can be 20 hours or shorter, in particular 10 hours or shorter.

When the hydrogen donor is liquid, the hydrogen donor can be used as the reaction solvent for the reaction, in general. It is also possible to use, as an auxiliary solvent, one of or a mixture of non-hydrogen-donating solvents such as toluene, tetrahydrofuran, acetonitrile, dimethylformamide, dimethyl sulfoxide, acetone, and methylene chloride, in order to dissolve the raw materials. In a case where a formic acid salt is used or in similar cases, it is also possible to carry out the reaction of a two-layer system by using water as an auxiliary solvent to dissolve the formic acid salt, in combination with an organic solvent. In this case, a phase transfer catalyst may be used in combination to accelerate the reaction. In addition, when hydrogen gas is used, the solvent is preferably an alcohol solvent such as methanol, ethanol, isopropanol, trifluoroethanol, or hexafluoro-2-propanol.

The reaction solvent is not particularly limited, as long as the solvent does not inhibit the reaction. Specific examples of the reaction solvent include hydrocarbons such as pentane, hexane, heptane, and cyclohexane; esters such as methyl acetate, ethyl acetate, and butyl acetate; aromatic hydrocarbons such as benzene, toluene, and xylene; nitriles such as acetonitrile; ethers such as diethyl ether, diisopropyl ether, methyl cyclopentyl ether, tetrahydrofuran, and dioxane; amides such as N,N-dimethylformamide and N,N-dimethylacetamide; dimethyl sulfoxide; and halogenated hydrocarbons such as methylene chloride, chloroform, chlorobenzene, and 1,2-dichloroethane. The reaction solvent is preferably heptane, cyclohexane, methyl acetate, ethyl acetate, butyl acetate, toluene, acetonitrile, tetrahydrofuran, dioxane, N,N-dimethylformamide, methylene chloride, or chlorobenzene, and more preferably tetrahydrofuran, dioxane, methyl acetate, ethyl acetate, or butyl acetate.

When a mixture solvent of two or more of these solvents is used, the mixing ratio is not particularly limited. The amount of the solvent used can be selected as appropriate depending on the reaction conditions, and the like. If necessary, the reaction is carried out with stirring.

Furthermore, to increase anti:sin ratios in the present reaction, adding dropwise a solution of a substrate to a solution of a catalyst for a long time is effective. In this case, a solvent in which the catalyst is dissolved can be the same as the above mentioned solvents. Additionally, the formic acid and amines which are used in the reaction may be added into the solution of the catalyst previously. Also, the formic acid and amines may be mixed with the solution of the substrate to add dropwise the substrate together with the formic acid and amines to the solution of the catalyst. A time required for adding dropwise the substrate, which is a time required for the completion of the reaction, is, but not be limited to, from 1 hour to 60 hours, preferably 5 hours to 15 hours.

The concentration of the ß-keto-α-aminocarboxylic acid ester represented by general formula (2) in the reaction solution is, for example, 100 to 0.1%(w/v), and preferably 100 to 10%(w/v), but is not particularly limited. In addition, the concentration can be changed as appropriate depending on the reaction solvent used.

After completion of the reaction, the target ß-hydroxy-α-aminocarboxylic acid ester can be obtained by one of or an appropriate combination of ordinary employed purification methods such as extraction, filtration, crystallization, distillation, and various kinds of chromatography.

Note that, after completion of the reaction asymmetric reduction, the target ruthenium complex can be separated by an ordinary crystallization technique such as concentration of the reaction liquid or addition of a poor solvent. In addition, when a hydrogen halide salt is by-produced in the above-described preparation, a washing operation with water may be conducted, if necessary. In this case, the operation can be carried out easily, because the amount of the ruthenium complex represented by general formula (1) and used in the present invention is very small.

### Examples

Hereinafter, the present invention will be described in detail on the basis of Examples. However, the present invention is not limited to these Examples.

Note that the NMR spectra used for identification and purity determination of complexes in the following Examples and the like were measured with a Mercury Plus 300 4 n model apparatus manufactured by Varian Technologies Japan Ltd., or Bruker BioSpin Avance III 500 System. Meanwhile, the GC analyses were conducted by using Chirasil-DEX CB (0.25 mm x 25 m, 0.25 µm) (manufactured by Varian, Inc.) or InertCapPure-WAX (0.25 mm × 30 m, 0.25 µm) (manufactured by GL Sciences Inc.). The HPLC analyses were conducted by using CHIRALCEL OJ-H (0.46 mm × 25 cm) (manufactured by Daicel Chemical Industries, Ltd.), ODS-3V (4.6 mm × 25 cm, 5 µm) (GL Sciences Inc.), or CHIRALPAK AD (4.6 mm × 25 cm).

Note that abbreviations in Examples have the following meanings.
TsDPEN: N-(p-toluenesulfonyl)-1,2-diphenylethylenediamine;
TIPPsDPEN:
N-(2,4,6-triisopropylbenzenesulfonyl)-1,2-diphenylethylenediamine o-TFTsDPEN:
N-(2-trifluoromethylmethylbenzenesulfonyl)-1,2-diphenylethylenediamine MESsDPEN:
N-(2,4,6-trimethylbenzenesulfonyl)-1,2-diphenylethylenediamine
   TsCYDN: N-(p-toluenesulfonyl)-1,2-cyclohexanediamine
   DIPEA: diisopropylethylamine
   DPPE: diphenylphosphinoethane
Note that the diamines in complexes represent those from which one or two hydrogen atoms were eliminated.
S/C represents a value of the number of moles of the substrate/the number of moles of the catalyst.

The following Synthesis 1 to Synthesis 9 show synthesis methods for obtaining ruthenium complexes represented by general formula (1).

### [Synthesis 1]

### Production of N-((1R,2R)-1,2-diphenyl-2-(2-(tetrahydro-2H-pyran-2-yloxy)ethylamino)ethyl)-4-methylbenzenesulfonamide

The target compound (B) was produced by the following reaction.

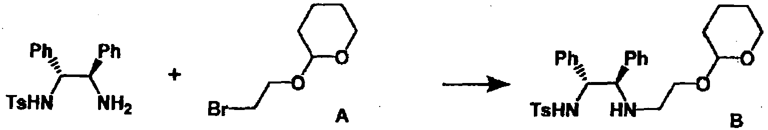

In a 50-ml Schlenk tube, 5.0 g (13.65 mmol) of (R,R)-TsDPEN and 2.85 g (2.07 ml) (13.65 mmol) of alkyl bromide (A) were mixed with each other in 10 ml of DMSO, and the reaction was allowed to proceed at 60°C for 29 hours. Then, 50 ml of dichloromethane and 50 ml of a saturated aqueous NaHCO₃ solution were added to the reaction mixture. After stirring, the organic layer was separated, and further washed twice with 50 ml of a saturated aqueous NaHCO₃ solution. The dichloromethane was recovered, and the residue was purified by silica gel column chromatography. Thus, 4.94 g of the target compound (B) was obtained. Yield: 72%.
¹H-NMR (CDCl₃, 300 MHz) δ:
1.43-1.80 (m, 6H), 2.32 (s, 3H), 2.42-2.70 (m, 2H), 3.40-3.55 (m, 2H), 3.70-3.85 (m, 2H), 3.77 (d, 1H), 4.30 (m, 1H), 4.45 (d, 1H), 6.93-7.38 (m, 14H)

### [Synthesis 2]

### Production of N-((1R,2R)-2-(2-hydroxyethylamino)-1,2-diphenylethyl)-4-methylbenzenesulfona mide

The target diamine (C) was produced by the following reaction.

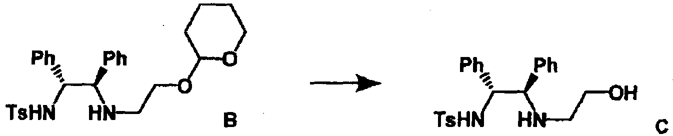

To 5.69 g of the above-described compound (B) obtained in Synthesis 1, 135 ml of ethanol and 34.5 ml of a 1 M aqueous HCl solution were added, and the reaction was allowed to proceed at 40°C for 2 hours. Then, the reaction mixture was neutralized by adding 3.45 g of NaHCO₃ thereto. Then, 75 ml of water and 150 ml of diethyl ether were added to the solution, and phase separation was conducted. Then, 50 ml of water was added thereto, and ether was removed with an evaporator. As a result, white crystals were precipitated. After the mixture was ice cooled, the crystals were filtered, washed with water, and then dried under reduced pressure at 70°C. Thus, 4.33 g of the target diamine (C) was obtained. Yield: 92%.
¹H-NMR (CDCl₃, 300 MHz) δ:
2.31 (s, 3H), 2.50-2.62 (m, 2H), 3.58-3.75 (m, 2H), 3.79 (d, 1H), 4.40 (d, 1H), 6.82-7.41 (m, 14H)

### [Synthesis 3]

### Production of (4-methylcyclohexa-1,4-dienyl)methanol

The target compound (F) was produced by the following reaction.

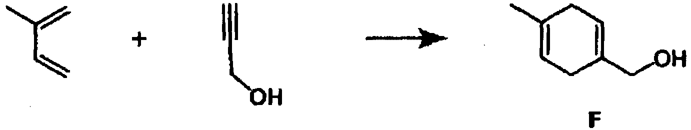

In a 500 mL four-necked flask, 1.73 g (7.93 mmol) of CoBr₂, 8.4 g (26.3 mmol) of ZnI₂, 3.47 g (8.8 mmol) of DPPE, and 370 ml of dichloromethane were placed. After nitrogen substitution, the mixture was stirred at 30°C for 30 minutes. Then, 78 ml (53.1 g, 780 mmol) of isoprene, 41 ml (39.3 g, 701 mmol) of propargyl alcohol, and 2.2 g (8.53 mmol) of Bu₄NBH₄ were added thereto, and the reaction was allowed to proceed at 30°C for 7 hours. Then, the dichloromethane solution was recovered, and the residue was distilled under reduced pressure at 160°C. Thus, 27.7 g of the target diene mixture (F) was obtained (32% yield). The purity of the target diene in this mixture was approximately 98% based on gas chromatography (GC).
¹H-NMR (CDCl₃, 300 MHz) δ:
1.67 (s, 3H), 2.55-2.70 (m, 4H), 4.02 (s, 2H), 5.44 (m, 1H), 5.68 (m, 1H)

### [Synthesis 4]

### Production of [RuCl₂ (1-(bromomethyl)-4-methylbenzene)]₂)

The target complex compound (G) was produced by the following reaction.

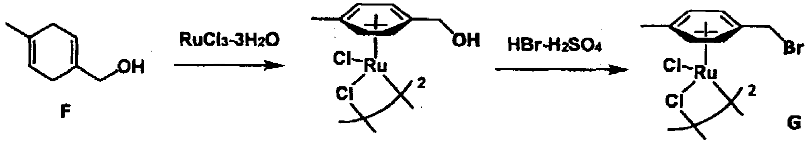

In 40 ml of 2-methoxyethanol and 4 ml of water, 4.75 g (38.2 mmol) of the above-described diene (F) obtained in Synthesis 3, 2.0 g (7.65 mmol) of ruthenium trichloride trihydrate, and 0.643 g (7.65 mmol) of NaHCO₃ were dissolved, and the reaction was allowed to proceed at 130°C for 1.5 hours. After that, the solvent was removed by distillation with an evaporator. To the residue, 52 ml of a concentrated aqueous hydrobromic acid solution and 4 ml of concentrated sulfuric acid were added, followed by stirring at 100°C for 4 hours. After the reaction, dichloromethane, water, and 2-methoxyethanol were added to the solution. The mixture was stirred, and allowed to stand. Then, the precipitated crystals were filtered. Thus, 1.9 g of the target complex (G) was obtained. Yield:79%.
¹H-NMR (DMSO-d₆, 300 MHz) δ:
2.23 (s, 3H), 4.40 (s, 2H), 5.84 (d, 2H), 6.15 (d, 2H)

### [Synthesis 5]

### Production of RuCl((R,R)-O-HT-TsDPEN)

The target complex RuCl((R,R)-O-HT-TsDPEN) was produced by the following reaction.

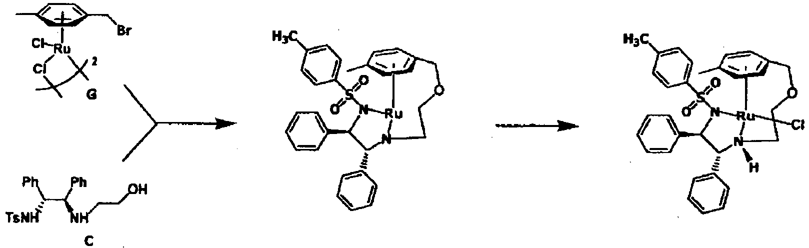

The above-described arene dimer (G) (1.6 g, 2.24 mmol) obtained in Synthesis 4, 1.53 g (3.73 mmol) of diamine (C) produced in Synthesis 2, 1.19 g (3.73 mmol) of triethylbenzylammonium iodide (Et₃BnNI), 52.8 ml of dichloromethane, and 52.8 ml of water were mixed with each other, and stirred at 35°C. To this mixture, 1.78 g (26.9 mmol) of KOH was added, and the reaction was allowed to proceed for 3 hours. The organic layer turned to be a violet solution. After the mixture was allowed to stand, the aqueous layer was removed. To the organic layer, 50 ml of water was added. The mixture was stirred, and allowed to stand, followed by phase separation. This phase separation operation was conducted three times. Thereafter, 65 ml of a 0.1 M aqueous HCl solution was added thereto, followed by stirring for 30 minutes. Then, the solution was neutralized by adding 0.034 g of NaHCO₃, and the mixture was allowed to stand. The dichloromethane layer alone was separated, and evaporated to dryness. The residue was purified with a silica gel column (eluent: CHCl₃/MeOH=20/1). Thus, 1.1 g of the target complex RuCl((R,R)-O-HT-TsDPEN) was obtained. Yield: 45% (the purity was approximately 95% based on liquid chromatography (HPLC)).
¹H-NMR (CD₂Cl₂, 300 MHz) δ:
2.25 (s, 3H), 2.52 (s, 3H), 3.13 (m, 1H), 3.60 (m, 1H), 3.80-4.00 (m, 4H), 4.48 (d, J=15.0 Hz, 1H), 4.52 (brs, 1H), 4.95 (d, J=15.0 Hz, 1H), 5.45 (d, J=5.2 Hz, 1H), 5.75 (d, J=6.2 Hz, 1H), 6.05 (d, J=5.2 Hz, 1H), 6.60 (d, J=6.9 Hz, 2H), 6.65-6.70 (m, 4H), 6.88 (d, J=8.0 Hz, 2H), 7.08-7.18 (m, 4H), 7.23 (d, J=8.0 Hz, 2H) HRMS (ESI):
   [M-C1]⁺ calcd for C₃₁H₃₃N₂O₃RuS: 615.1258; found: 615.1258

### [Synthesis 6]

### Production of 2-((4-methylcyclohexa-1,4-dienyl)methoxy)ethanol and 2-((5-methylcyclohexa-1,4-dienyl)methoxy)ethanol

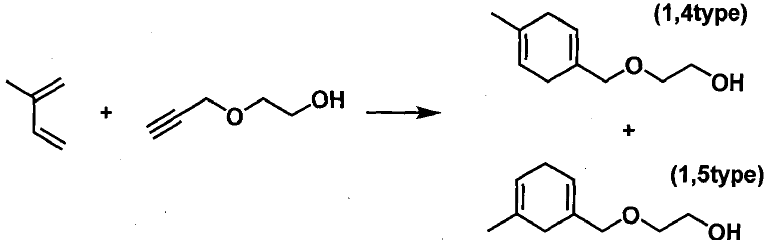

In 460 ml of THF, 7.74 g (0.019 mol) of 1,2-bis(diphenylphosphino)ethane, 4.05 g (0.019 mol) of cobalt bromide, 11.82 g (0.037 mol) of zinc iodide, and 2.42 g (0.037 mol) of zinc were dissolved, and the mixture was stirred at 70°C for 15 minutes. After cooling to room temperature, 74.89 g (1.10 mol) of isoprene was added thereto, and then 92.70 g (0.93 mol) of an alkyne alcohol was added dropwise slowly, with cooling in a water bath. After stirring at 35°C for 1 hour, the solvent was removed by distillation under reduced pressure. To the obtained residue, 460 ml of toluene and 460 ml of water were added (stirring: 10 minutes, allowing to stand: 10 minutes). Under nitrogen atmosphere, filtration was conducted through Celite, and then the obtained solution was phase separated. The solvent was removed by distillation under reduced pressure. The obtained crude product was purified by Claisen distillation (101 to 113°C/3 torr). Thus, 106.6 g of the diene alcohols were obtained as a colorless oily substance. Yield: 68.5% (1,4-type/1,5-type=91/9).
¹H-NMR (CDCl₃, 300 MHz) δ:
1.68 (s, 3H), 2.31 (brs, 1H), 2.64 (brs, 4H), 3.48-3.52 (m, 2H), 3.70-3.75 (m, 2H), 3.93 (s, 2H), 5.43-5.45 (m, 1H), 5.70-5.71 (m, 1H);
   HRMS(ESI):
   [M+H]⁺ calcd for C₁₀H₆O₂: 167.1430; found: 167.1432

### [Synthesis 7]

Production of 2-((4-methylcyclohexa-1,4-dienyl)methoxy)ethyl 4-methylbenzenesulfonate and 2-((5-methylcyclohexa-1,4-dienyl)methoxy)ethyl 4-methylbenzenesulfonate

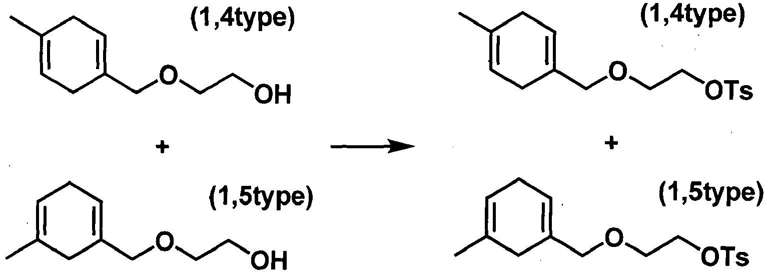

In 400 ml of toluene, 100.00 g (0.59 mol) of the diene alcohols obtained in Synthesis 6, 90.29 g (0.89 mol) of triethylamine, 73.20 g (0.89 mol) of 1-methylimidazole were dissolved. To this solution cooled in an ice bath, a toluene solution (400 ml) of 130.33 g (0.68 mol) of p-toluenesulfonyl chloride was added dropwise slowly, followed by stirring at room temperature for 1 hour. Water was added thereto, and phase separation was conducted. The obtained organic layer was washed with 15% sulfuric acid, water, and saturated aqueous sodium hydrogen carbonate in this order. The solvent was removed by distillation under reduced pressure. Thus, 188.01 g of the target tosylates were obtained as a colorless oily substance. Yield: 98.1% (1,4-type/1,5-type=91/9).
¹H-NMR (CDCl₃, 300 MHz) δ:
1.67 (s, 3H), 2.44 (s, 3H), 2.58 (brs, 4H), 3.58-3.55 (m, 2H), 3.84 (s, 2H), 4.18-4.14 (m, 2H), 5.41-5.40 (m, 1H), 5.64-5.63 (m, 1H), 7.33 (d, J=8.3 Hz, 1H), 7.80 (d, J=8.3 Hz, 1H);
   HRMS (ESI):
   [M+H]⁺ calcd for C₁₇H₂₂O₄S: 323.1312; found: 323.1325

### [Synthesis 8]

### Production of 2,4,6-triisopropyl-N-((1R,2R)-2-(2-((4-methylcyclohexa-1,4-dienyl)methoxy)etheyl amino)-1,2-diphenylethy)benzenesulfonamide

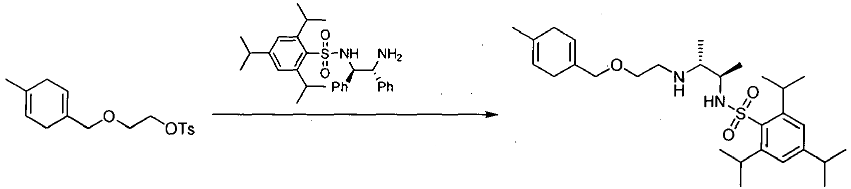

In 25 ml of toluene, 6.03 g (18.82 mmol) of the above-described tosylates obtained in Synthesis 7 were dissolved. To this solution, 2.43 g (18.82 mmol) of DIPEA and 9.00 g (18.80 mmol) of (R,R)-TIPPsDPEN were added, followed by stirring at 135°C for 13 hours. After that, the solvent was removed by distillation under reduced pressure. The obtained residue was purified by silica gel column chromatography (toluene/ethyl acetate=20/1→+15/l). Thus, 10.53 g of the title compound was obtained as a colorless oily substance. Yield: 89.0%.
¹H-NMR (CDCl₃, 300 MHz) δ:
1.06 (d, J=6.9 Hz, 3H), 1.21 (d, J=6.9 Hz, 3H), 1.87 (brs, 1H), 1.68 (s, 3H), 2.60 (brs, 4H), 2.71-2.48 (m, 2H), 3.52-3.34 (m, 2H), 3.55 (d, J=8.9 Hz, 1H), 3.77 (s, 2H), 3.95 (septet, J=6.7 Hz, 3H), 4.40 (d, J=8.9 Hz, 1H), 5.44 (m, 1H), 5.64 (m, 1H),6.52 (brs, 1H), 6.74-7.28 (m, 12H);
   HRMS(ESI):
   [M+H]⁺ calcd for C₃₉H₅₃N₂O₃S: 629.3771; found: 629.3771

### [Synthesis 9]

### Production of RuCl((R,R)-O-HT-TIPPsDPEN)

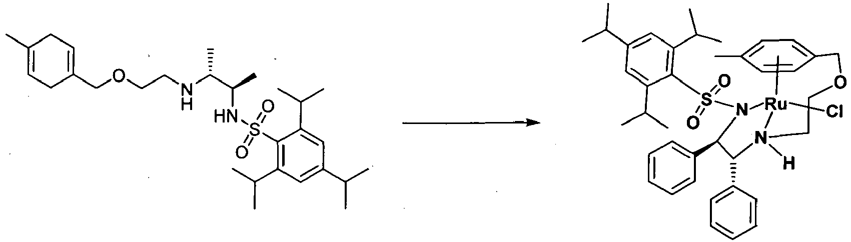

In 8 ml of methanol, 2.02 g (3.21 mmol) of the above-described sulfonamide obtained in Synthesis 8 was dissolved. Under ice-cooling, 0.67 g (6.42 mmol) of a 1 M hydrochloric acid solution in methanol was added thereto, followed by stirring at room temperature for 20 minutes. Then, the solvent was removed by distillation under reduced pressure. The obtained residue was dissolved in 30 ml of 3-methoxypropanol and 18 ml of water. To this solution, 0.72 g (2.75 mmol) of ruthenium trichloride trihydrate was added, followed by stirring at 120°C for 1 hour. The solvent was removed by distillation under reduced pressure. To the obtained residue, 35 ml of IPA and 0.72 g (7.15 mmol) of triethylamine were added, followed by stirring at 60°C for 1 hour. The solvent was removed by distillation under reduced pressure. The obtained residue was purified by silica gel column chromatography (chloroform/methanol=97/3→20/1). Thus, 1.28 g of the target Ru complex was obtained. Yield:52.3%.
¹H-NMR (CD₂Cl₂, 500 MHz) δ:
1.0-1.2 (m, 18H), 1.70 (m, 1H), 2.41 (s, 3H), 2.60 (m, 1H), 3.05 (m, 1H), 3.35 (m, 1H), 3.68 (m, 1H), 3.75 (t, 1H), 3.85 (m, 2H), 4.18 (d, 1H), 4.25 (d, 1H), 4.85 (brs, 1H), 5.02 (d, 1H), 5.30 (d, 1H), 5.48 (d, 1H), 5.63 (d, 1H), 6.35 (d, 1H), 6.40-6.70 (m, 10H), 6.90-7.05 (m, 3H);
   HRMS (ESI):
   [M+H]⁺ calcd for C₃₉H₅₀N₂O₃SClRu: 763.2269; found: 763.2257

### [Synthesis 10]

### Production of N-((1R,2R)-2-(2-((4-methylcyclohexa-1,4-dienyl)methoxy)ethylamino)-1,2-diphen ylethyl)-2-(trifluoromethyl)benzenesulfonamide hydrochloride

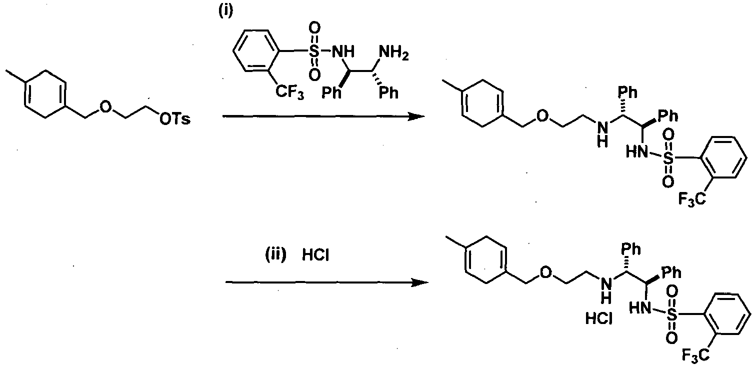

In 31.6 ml of toluene, 8.07 g (26.1 mmol) of the above-described tosylates obtained in Synthesis 7 were dissolved. To this solution, 3.38 g (26.2 mmol) of DIPEA, 10.00 g (23.8 mmol) of (R,R)-o-TFTsDPEN and 4.34 g (26.2 mmol) of potassium iodide were added, followed by stirring at 135°C for 6 hours. The reaction solution was concentrated and purified with a silica gel column chromatography to obtain 10.1 g of diamine (Yield: 74.5 %). Then, 110 ml of dichloromethane and 65.3 ml of HCl in methanol solution (1N) were added to 10.1 g (17.7 mmol) of the diamine, followed by stirring for 0.5 hours, and the solvent was removed to obtain 11.1 g of the target diamine hydrochloride. Yield: 93.9%
¹H-NMR(DMSO,300MHz) δ:
1.62(m, 3H), 2.60(s, 3H), 2.78-3.12(m, 2H), 3.52-3.70(m, 2H), 3.86(s, 2H) ,4.75(m, 1H), 4.92(m, 1H), 5.40(m, 1H), 5.68(m, 1H), 6.75-7.35(m, 10H), 7.40(t, 1H), 7.50(t, 1H), 7.60(d, 1H), 7.75(d, 1H), 8.90(m, 1H), 8.98(brd, 1H), 9.92(brd, 1H); ¹⁹F-NMR(DMSO) δ:
   - 57.16;
      HRMS (ESI):
      [M-Cl]⁺ calcd for C₃₁H₃₃N₂O₃F₃S·HCl: 571.2237; found: 571.2244

### [Synthesis 11]

### Production of RuCl((R,R)-O-HT-o-TFTs-DPEN)

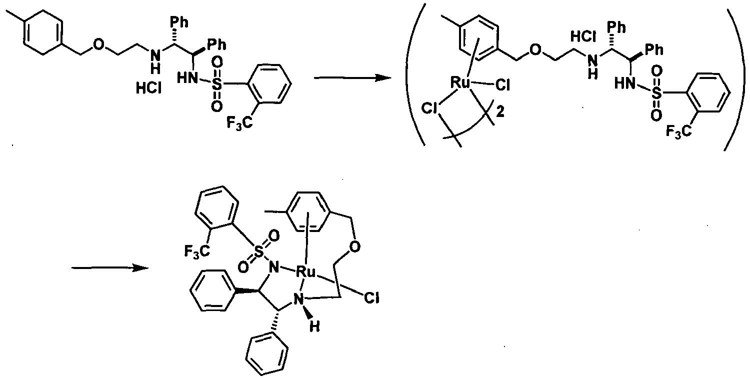

In 66 ml of 3-methoxypropanol and 22 ml of water, 5.0 g (8.25 mmol) of the diamine hydrochloride obtained in Synthesis 10 was dissolved. After that, to the solution, 1.79 g (6.86 mmol) of rutheniumchloride trihydrate and 0.58 g (6.86 mmol) of sodium hydrogencarbonate were added, followed by stirring at 120°C for 2 hours. After collecting 50 ml of 3-methoxypropanol from the solution, to the solution, 75 ml of MIBK and 2.78 g (27.45 mmol) of triethylamine were added, followed by stirring at 60°C for 1 hour. Then, a 0.3 M hydrochloric acid was added to the solution, and phase separation was conducted, followed by washing an obtained organic phase with water twice. After about 60 ml of the solvent was collected, 85 ml of heptane was added, and precipitation was conducted. The precipitated crystals were filtered, and then 4.60 g of the target Ru complex was obtained. Yield: 95.2%.
¹H-NMR(CD₂Cl₂,300MHz) δ:
2.50 (s, 3H), 3.15 - 3.20 (m, 1H), 3.70 - 3.82 (m, 2H), 4.00 (m, 2H), 4.15 (m, 1H), 4.40 (m, 1H), 4.80 (m, 1H), 5.10 (d, 1H), 5.45 (d, 1H), 5.62 (d, 1H), 5.70 (d, 1H), 6.38 (d, 1H), 6.50-7.50(m, 14H);
   ¹⁹F-NMR(DMSO) δ:
   - 58.45
      HRMS(ESI):
      [M+H]⁺ calcd for C₃₁H₃₀ClN₂O₃F₃RuS: 705.7034; found: 705.0758

### [Synthesis 12]

### Production of 2,4,6-trimethyl-N-((1R,2R)-2-(2-((4-methyl cyclohexa-1,4-dienyl)methoxy)ethylamino)-1,2-diphenylethyl)benzenesulfonamid e

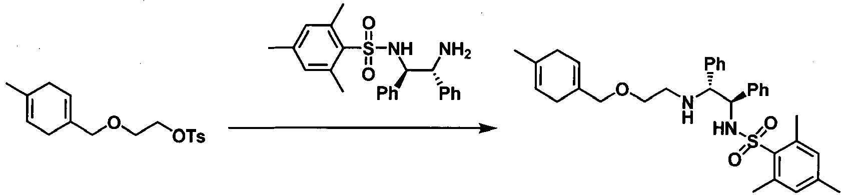

In 5 ml of toluene, 1.0 g (3.0 mmol) of the above-described tosylates obtained in Synthesis 7 was dissolved. To this solution, 0.39 g (3.0 mmol) of DIPEA and 1.3 g (3.3 mmol) of (R,R)-MESsDPEN were added, followed by stirring at 120°C for 8 hours. After that, the solvent was removed by distillation under reduced pressure. The obtained residue was purified with a silica gel column chromatography (toluene/ethyl acetate=4/1). Thus, 0.71 g of the title compound was obtained as a colorless oily substance. Yield: 44.7 %.

### [Synthesis 13]

### Production of RuCl((R,R)-O-HT-MESs-DPEN)

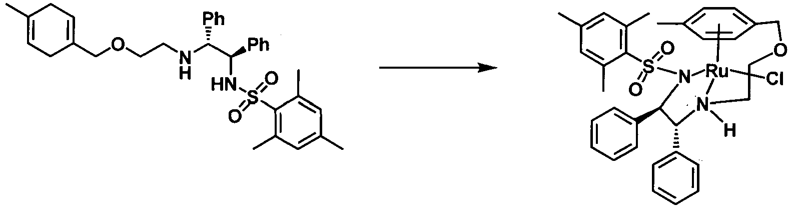

In 5 ml of methanol, 0.67 g (1.2 mmol) of the above-described sulfonamide obtained in Synthesis 12 was dissolved. Under ice-cooling, 0.25 g (2.4 mmol) of a 1 M hydrochloric acid solution in methanol was added thereto, followed by stirring at room temperature for 20 minutes. Then, the solvent was removed by distillation under reduced pressure. The obtained residue was dissolved in 20 ml of 2-methoxyethanol, 2 ml of water and 0.09 g (1.2 mmol) of sodium hydrogencarbonate. To this solution, 0.36 g (1.35 mmol) of rutheniumchloride trihydrate was added, followed by stirring at 120°C for 3 hours. The solvent was removed by distillation under reduced pressure. To the obtained residue, 40 ml of ethanol, 0.5 g (4.94 mmol) of triethylamine were added, followed by stirring at 80°C for 2 hour. The solvent was removed by distillation under reduced pressure. The obtained residue was purified with a silica gel column chromatography (chloroform/methanol=20/1). Thus, 0.13 g of the target Ru complex was obtained. Yield: 16.0 %.
¹H-NMR(CD₂Cl₂,500MHz) δ:
1.95 (s, 3H), 2.45(s, 6H), 2.46(s, 3H), 3.05(m, 1H), 3.70(m, 1H), 3.80(d, 1H), 3.85(m, 2H), 3.95(d, 1H), 4.25(d, 1H), 4.75(m, 1H), 5.00(d, 1H), 5.40(d, 1H), 5.50(d, 1H), 5.60(d, 1H), 6.30(s, 2H), 6.53(d, 1H), 6.40-7.00(m, 10H);
   HRMS(ESI):
   [M+H]⁺ calcd for C₃₃H₃₇ClN₂O₃RuS: 679.1335; found: 679.1327

### [Synthesis 14]

### Production of 4-methyl-N-((1R,2R)-2-(2-((4-methyl cyclohexa-1,4-dienyl)methoxy)ethylamino)cyclohexyl)benzenesulfonamide hydrochloride

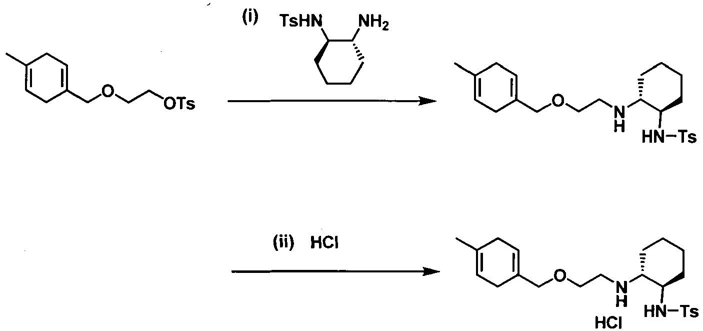

In 26 ml of toluene, 5.06 g (16.4 mmol) of the above-described tosylates obtained in Synthesis 7 were dissolved. To this solution, 2.12 g (16.4 mmol) of DIPEA, 4.00 g (14.9 mmol) of (R,R)-TsCYDN and 2.72 g (16.4 mmol) of potassium iodide were added, followed by stirring at 135°C for 20 hours. The reaction solution was concentrated and purified with a silica gel column to obtain 2.9 g of diamine (Yield: 46.9 %). Then, 42 ml of dichloromethane and 24.6 ml of HCl in methanol solution (1N) were added to 2.8 g (6.69 mmol) of the diamine, followed by stirring for 0.5 hours, and the solvent was removed to obtain 2.9 g of the target diamine hydrochloride. Yield: 94.7 %
¹H-NMR(DMSO, 300MHz) δ:
0.95-1.30(m, 4H), 1.50(m, 2H), 1.63(s, 3H), 2.10(m, 2H), 2.40(s, 3H), 2.60(m, 2H), 2.95(brd, 1H), 3.18(m, 2H), 3.60(m, 2H), 3.90(s, 2H), 5.40(m, 1H), 5.70(m, 1H), 7.40(d, 1H), 7.75(d, 1H), 8.15(d, 1H), 8.23(brd, 1H), 9.10(brd, 1H)
   HRMS(ESI):
   [M-Cl]⁺ calcd for C₂₃H₃₄N₂O₃S: 419.2363; found: 419.2365

### [Synthesis 15]

### Production of RuCl((R,R)-O-HT-Ts-cydn)

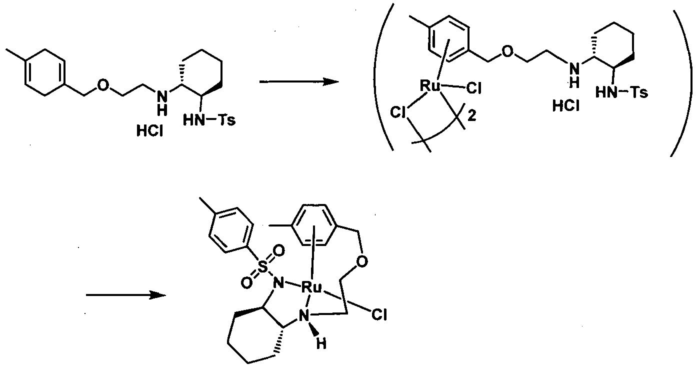

In 15 ml of 3-methoxypropanol and 3 ml of water, 0.5 g (1.1 mmol) of the diamine hydrochloride obtained in Synthesis 14 was dissolved. After that, to the solution, 0.25 g (0.96 mmol) of rutheniumchloride trihydrate and 0.08 g (0.96 mmol) of sodium hydrogencarbonate were added, followed by stirring at 120°C for 1 hour. After collecting 12 ml of 3-methoxypropanol from the solution, to the solution, 13 ml of MIBK and 0.39 g (3.82 mmol) of triethylamine were added, followed by stirring at 60°C for 1 hour. Then, a 0.3 M hydrochloric acid was added to the solution, and phase separation was conducted, followed by washing an obtained organic phase with water twice. After about 10 ml of the solvent was collected, 15 ml of heptane was added, precipitation was conducted. The precipitated crystals were filtered, and then 0.24 g of the target Ru complex was obtained. Yield: 45.5 %.
¹H-NMR(CD₂Cl₂,500MHz) δ:
0.65-1.05 (m, 4H), 1.90 (m, 1H), 1.15 (m, 1H), 2.08 (m, 1H), 2.70 (m, 1H), 2.75 (s, 1H), 2.77 (s, 1H), 2.60 (m, 1H), 3.60-3.70 (m, 2H), 3.80 (m, 1H), 4.00 (m, 1H), 4.25 (m, 1H), 4.35 (d, 1H), 4.92 (d, 1H), 5.25 (d, 1H), 5.50 (d, 1H), 5.67 (d, 1H),5.83 (d, 1H), 7.20 (d, 1H), 7.80 (d, 1H);
   HRMS(ESI):
   [M-Cl]⁺ calcd for C₂₃H₃₁N₂O₃RuS: 517.1093; found: 517.1101

### [Example 1]

### Production of methyl (2R,3R)-2-acetylamino-3-hydroxyoctadecanoate

To a solution of RuCl((R,R)-O-HT-TsDPEN) (6.5 mg, 0.01 mmol) produced in Synthesis 5 and methyl 2-acetylamino-3-oxooctadecanoate (0.93 g, 2.50 mmol) in 1,4-dioxane (4.6 ml), triethylamine (0.758 g, 7.5 mmol) and formic acid (0.345 g, 7.5 mmol) were added. Then, the mixture was heated to 90°C, and stirring was continued for 6 hours. A sample was taken out, and anaylzed by HPLC. As a result, the conversion was 99%. In addtion, the ratio between the anti isomer ((2R,3R) isomer) and the syn isomer((2S,3R) isomer) was as followes: anti isomer:syn isomer=90.5:9.5.

After cooling to 20°C, the reaction liquied was washed wtih water, and drived over magnesium sulfate. The solvent was recovered under reduced pressure. Thus, the titled compound was obtained. The obtained crude product was analyzed by cmparison with a standard substance. As a result, the content of the titled compound was 0.88 g, and the yield thereof was 95%. In addtion, the optical purity of the anti isomer was 97%ee.

### [Example 2]

### Production of methyl (2R,3R)-2-acetylamino-3-hydroxyoctadecanoate

To a solution of RuCl((R,R)-O-HT-TIPPsDPEN) (7.6 mg, 0.01 mmol) produced in Synthesis 9 and methyl 2-acetylamino-3-oxooctadecanoate (0.93 g, 2.50 mmol) in 1,4-dioxane (4.6 ml), triethylamine (0.758 g, 7.5 mmol) and formic acid (0.345 g, 7.5 mmol) were added. The mixture was heated to 90°C, and stirring was continued for 6 hours. A sample was taken out, and analyzed by HPLC. As a result, the conversion was 70%. In addition, the ratio between the anti isomer ((2R,3R) isomer) and the syn isomer ((2S,3R) isomer) was as follows: anti isomer:syn isomer=92.5:7.5. In addition, the optical purity of the anti isomer was 97%ee.

### [Comparative Example]

### Production of methyl (2R,3R)-2-acetylamino-3-hydroxyoctadecanoate

To a solution of RuCl[(R,R)TsDPEN)](p-cymene) complex (6.4 mg, 0.01 mmol) and methyl 2-acetylamino-3-oxooctadecanoate (0.93 g, 2.50 mmol) in 1,4-dioxane (4.6 ml), triethylamine (0.758 g, 7.5 mmol) and formic acid (0.345 g, 7.5 mmol) were added. The mixture was heated to 90°C, and stirring was continued for 6 hours. A sample was taken out, and analyzed by HPLC. As a result, the conversion was 65%. In addition, the ratio between the anti isomer ((2R, 3R) isomer) and the syn isomer ((2S, 3R) isomer) was as follows: anti isomer:syn isomer=81.5:18.5. In addition, the optical purity of the anti isomer was 97%ee.

Table 1 shows the results of Examples 1 and 2, as well as Comparative Example.

**Table 1**

| Example | Catalyst | s/c | Temp. /°C | Time /hour | Conv. /% | anti:syn | De /%de |
|---|---|---|---|---|---|---|---|
| 1 | RuCl((R,R)-O-HT-TsDPEN) | 250 | 90 | 6 | >99 | 90.5:10.5 | 81 |
| 2 | RuCl((R,R)-O-HT-TIPPsDPEN) | 250 | 90 | 6 | 70 | 92.5:7.5 | 85 |
| (Comp. Ex.) | RuCl[(R,R)TsDPEN)(p-cymene)] | 250 | 90 | 6 | 65 | 81.5^{:}18.5 | 63 |

Here, De in this specification represents the mass ratio of (anti isomer-syn isomer)/(anti isomer+syn isomer).

From the contents of Examples 1 and 2 and Comparative Example, it was found that when the reaction conditions (s/c, temperature, and reaction time) were the same, the conversion was remarkably increased by selecting the ruthenium complex used for the invention of the present application. In addition, it was also found that the anti isomer was obtained with high stereo selectivity.

### [Examples 3 to 5]

### Production of methyl (2R,3R)-2-acetylamino-3-hydroxyoctadecanoate

Different amines were added respectively to solutions each containing RuCl((R,R)-O-HT-TsDPEN) (6.5 mg, 0.01 mmol) produced in Synthesis 5 and methyl 2-acetylamino-3-oxooctadecanoate (0.93 g, 2.50 mmol) in tetrahydrofuran (4.6 ml). Then, formic acid (0.345 g, 7.5 mmol) was further added to this mixture, and stirring was continued at 30°C for 20 hours.

Table 2 shows the results.

**Table 2**

| Example | Catalyst | s/c | Amine | Amine /eq. | Temp. /°C | Time /hour | Conv. /% | anti:syn | De /%de |
|---|---|---|---|---|---|---|---|---|---|
| 3 | RuCl((R,R)-O-HT-TsDPEN) | 250 | Et₃N | 3 | 30 | 20 | 80 | 94.2:5.8 | 88.4 |
| 4 | RuCl((R,R)-O-HT-TsDPEN) | 250 | nBu₃N | 3 | 30 | 20 | 85 | 93.7:6.3 | 87.4 |
| 5 | RuCl((R,R)-O-HT-TsDPEN) | 250 | EtN(iPr)₂ | 3 | 30 | 20 | 95 | 94.9:5.1 | 89.8 |

| | | | | | | | | | |
|---|---|---|---|---|---|---|---|---|---|
| Et₃N: Triethylamine nBu₃N: Tri-n-butylamine EtN(iPr)₂: Diisopropylethylamine | | | | | | | | | |

Examples 3 to 5 showed that the use of tertiary organic amines made it possible to complete the reaction even under a mild temperature condition (30°C) in a relatively short reaction time (20 hours), and to achieve high ratios of anti:syn.

### [Examples 6 to 15]

### Production of methyl (2R,3R)-2-acetylamino-3-hydroxyoctadecanoate

Different amines were added respectively to solutions each containing RuCl((R,R)-O-HT-TsDPEN) (5.2 mg, 0.008 mmol) produced in Synthesis 5 and methyl 2-acetylamino-3-oxooctadecanoate (1.48 g, 4.00 mmol) in tetrahydrofuran (14.8 ml). Then, formic acid (0.552 g, 12.0 mmol) was further added to this mixture, and stirring was continued at 60°C for 20 hours.

Table 3 shows the results.

**Table 3**

| Example | Catalyst | s/c | Amine | Amine /eq. | Temp. /°C | Time /hour | Conv. /% | anti:syn | De /%de |
|---|---|---|---|---|---|---|---|---|---|
| 6 | RuCl((R,R)-O-HT-TsDPEN) | 500 | 2,6-Lutidine | 3 | 60 | 20 | 100 | 88.6:11.4 | 73.2 |
| 7 | RuCl((R,R)-O-HT-TsDPEN) | 500 | Morpholine | 3 | 60 | 20 | 79 | 84.8:15.2 | 69.6 |
| 8 | RuCl((R,R)-O-HT-TsDPEN) | 500 | 1-Me-pyrrolidine | 3 | 60 | 20 | >99 | 85.9:14.1 | 71.8 |
| 9 | RuCl((R,R)-O-HT-TsDPEN) | 500 | (i-Oct)₃N | 3 | 60 | 20 | 96 | 86.5:13.5 | 73.0 |
| 10 | RuCl((R,R)-O-HT-TsDPEN) | 500 | iPrMe₂N | 3 | 60 | 20 | 73 | 87.3:12.7 | 74.6 |
| 11 | RuCl((R,R)-O-HT-TsDPEN) | 500 | 1-Et-piperidine | 3 | 60 | 20 | >99 | 87.0:13.1 | 73.9 |
| 12 | RuCl((R,R)-O-HT-TsDPEN) | 500 | 1-Me-piperidine | 3 | 60 | 20 | 79 | 87.0:13.0 | 74.0 |
| 13 | RuCl((R,R)-O-HT-TsDPEN) | 500 | Cy₂MeN | 3 | 60 | 20 | 99 | 87.8:12.2 | 75.6 |
| 14 | RuCl((R,R)-O-HT-TsDPEN) | 500 | Diethylaniline | 3 | 60 | 20 | 94 | 86.6:13.4 | 73.2 |
| 15 | RuCl((R,R)-O-HT-TsDPEN) | 500 | (CH₃CHOH)₃N | 3 | 60 | 20 | >99 | 84.3:16.7 | 68.6 |

| | | | | | | | | | |
|---|---|---|---|---|---|---|---|---|---|
| 1-Me-pyrrolidine: 1-methylpyrrolidine (i-Oct)₃ N: tri-iso-octylamine iPrMe₂N: isopropyldimethylamine 1-Et-piperidine: 1-ethylpiperidine 1-Me-piperidine: 1-methylpiperidine Cy₂MeN: dicyclohexylmethylamine | | | | | | | | | |

From the results of Examples 6 to 15, it was found that the use of a primary, secondary or tertiary organic amine, made it possible to complete the reaction for a relatively short reaction time (20 hours) and to achieve relatively high anti:syn ratios by a somewhat higher temperature condition (60°C) than in Example 3 to 5, even if the amount of the catalysts was small (the substrate:catalyst (S/C) ratio was 500).

### [Example 16]

### Production of methyl (2R,3R)-2-acetylamino-3-hydroxyoctadecanoate

To a solution of RuCl(R,R)-O-HT-TsDPEN) (2.6 mg, 0.004 mmol) (S/C=1,000) produced in Synthesis 5 and methyl 2-acetylamino-3-oxooctadecanoate (1.48 g, 4.00 mmol) in ethyl acetate (14.8 ml), triethylamine (1.21 g, 12.0 mmol) was added. Furthermore, formic acid (0.552 g, 12.0 mmol) was added to the solution, and stirring was continued at 60°C for 20 hours. A sample was taken out, and anaylzed by HPLC. As a result, the conversion was 97%. In addtion, the ratio between the anti isomer ((2R,3R) isomer) and the syn isomer((2S,3R) isomer) was as followes: anti isomer:syn isomer=88.3:11.7.

### [Example 17]

### Production of methyl (2R,3R)-2-acetylamino-3-hydroxyoctadecanoate

To a solution of RuCl((R,R)-O-HT-TsDPEN) (2.6 mg, 0.004 mmol) (S/C=1,000) produced in Synthesis 5 and methyl 2-acetylamino-3-oxooctadecanoate (1.48 g, 4.00 mmol) in methyl acetate (14.8 ml), triethylamine (1.21 g, 12.0 mmol) was added. Furthermore, formic acid (0.552 g, 12.0 mmol) was added to the solution, and stirring was continued at 60°C for 20 hours. A sample was taken out, and anaylzed by HPLC. As a result, the conversion was 94%. In addtion, the ratio between the anti isomer ((2R,3R) isomer) and the syn isomer((2S,3R) isomer) was as followes: anti isomer:syn isomer=88.5:11.5.

From the results of Examples 16 and 17, it was found that the use of suitable solvents such as ethyl acetate and methyl acetate made it possible to complete the reaction for a relatively short reaction time (20 hours) and to achieve relatively high anti:syn ratios by a temperature condition of 60°C, even if a condition where the amount of the catalysts was very small (the substrate:catalyst (S/C) ratio was 1,000).

### [Example 18]

### Production of methyl (2R,3R)-2-acetylamino-3-hydroxyoctadecanoate

To a solution of RuCl((R,R)-O-HT-TsDPEN) (2.6 mg, 0.004 mmol) (S/C=1,000) produced in Synthesis 5 and methyl 2-acetylamino-3-oxooctadecanoate (1.48 g, 4.00 mmol) in ethyl acetate (14.8 ml), diisopropylethylamine (1.55 g, 12.0 mmol) was added. Furthermore, formic acid (0.552 g, 12.0 mmol) was added to the solution, and stirring was continued at 60°C for 20 hours. A sample was taken out, and anaylzed by HPLC. As a result, the conversion was 100%. In addtion, the ratio between the anti isomer ((2R,3R) isomer) and the syn isomer((2S,3R) isomer) was as followes: anti isomer:syn isomer=87.8:12.2 (75.7%de).

### [Example 19]

### Production of methyl (2R,3R)-2-acetylamino-3-hydroxyoctadecanoate

A solution of RuCl((R,R)-O-HT-TsDPEN) (2.6 mg, 0.004 mmol) (S/C=1,000) produced in Synthesis 5 and diisopropylethylamine (1.55 g, 12.0 mmol) in ethyl acetate (14.8 ml), was heated to 60°C and stirred. Then, to the solution, a solution of methyl 2-acetylamino-3-oxooctadecanoate (1.48 g, 4.00 mmol) and formic acid (0.552 g, 12.0 mmol) in 5 ml THF were added dropwise over 10 hours, and stirring was continued for 10 hours. A sample was taken out, and anaylzed by HPLC. As a result, the conversion was 100%. In addtion, the ratio between the anti isomer ((2R,3R) isomer) and the syn isomer((2S,3R) isomer) was as followes: anti isomer:syn isomer=91.7:8.3 (83.3% de).

Having reviewed Example 18 and Example 19, it was found that the slowly adding dropwise the solution of the substrate to the solution of the catalyst in the reaction of Example 19 made it possible to further increase anti:syn ratios, compared to the reaction of Example 18, in which all substrates are mixed with the solution of the catalyst from the start.

### [Example 20]

### Introduction of methyl (2R,3R)-2-acetylamino-3-hydroxyoctadecanoate

To a solution of RuCl((R,R)-O-HT-o-TFTs-DPEN) (2.8 mg, 0.004 mmol) (S/C=1,000) produced in Synthesis 11 and methyl 2-acetylamino-3-oxooctadecanoate (1.48 g, 4.00 mmol) in methyl acetate (14.8 ml), diisopropyl ethylamine (1.55 g, 12.0 mmol) was added. Furthermore, formic acid (0.552 g, 12.0 mmol) was added to the solution, and stirring was continued at 60°C for 20 hours. A sample was taken out, and anaylzed by HPLC. As a result, the conversion was 92%. In addtion, the ratio between the anti isomer ((2R,3R) isomer) and the syn isomer((2S,3R) isomer) was as followes: anti isomer:syn isomer=91.0:9.0 (82.0%de).

### [Example 21]

### Production of methyl (2R,3R)-2-acetylamino-3-hydroxyoctadecanoate

To a solution of RuCl((R,R)-O-HT-MESs-DPEN) (2.7 mg, 0.004 mmol) (S/C=1,000) produced in Synthesis 13 and methyl 2-acetylamino-3-oxooctadecanoate (1.48g, 4.00 mmol) in methyl acetate (14.8 ml), diisopropylethylamine (1.55 g, 12.0 mmol) was added. Furthermore, formic acid (0.552 g, 12.0 mmol) was added to the solution, and stirring was continued at 60°C for 20 hours. A sample was taken out, and anaylzed by HPLC. As a result, the conversion was 95%. Then, additionally stirring was continued for 20 hours, and a sample was taken out again, and anaylzed by HPLC. As a result, the conversion was 100%. In addtion, the ratio between the anti isomer ((2R,3R) isomer) and the syn isomer((2S,3R) isomer) was as followes: anti isomer:syn isomer=91.6:8.4 (83.3%de).

### [Example 22]

### Production of methyl (2R,3R)-2-acetylamino-3-hydroxyoctadecanoate

To a solution of RuCl((R,R)-O-HT-Ts-cydn) (2.7 mg, 0.004 mmol) (S/C=1,000) produced in Synthesis 15 and methyl 2-acetylamino-3-oxooctadecanoate (1.48 g, 4.00 mmol) in methyl acetate (14.8 ml), diisopropyl ethylamine (1.55 g, 12.0 mmol) was added. Furthermore, formic acid (0.552 g, 12.0 mmol) was added to the solution, and stirring was continued at 60°C for 20 hours. A sample was taken out, and anaylzed by HPLC. As a result, the conversion was 89%. Then, additionally stirring was continued for 20 hours, and a sample was taken out again, and anaylzed by HPLC. As a result, the conversion was 98%. In addtion, the ratio between the anti isomer ((2R,3R) isomer) and the syn isomer((2S,3R) isomer) was as followes: anti isomer:syn isomer=89.0:11.0 (78.1%de).

It is possible to selectively produce an optically active β-hydroxy-α-aminocarboxylic acid ester useful as a raw material for producing pharmaceuticals and functional materials and as the like.

## Claims

1. A method for producing an optically active β-hydroxy-α-aminocarboxylic acid ester, comprising performing an asymmetric reduction reaction of a β-keto-α-aminocarboxylic acid ester in the presence of a ruthenium complex and a hydrogen donor, wherein
the ruthenium complex is represented by the following general formula (1) or (1)': 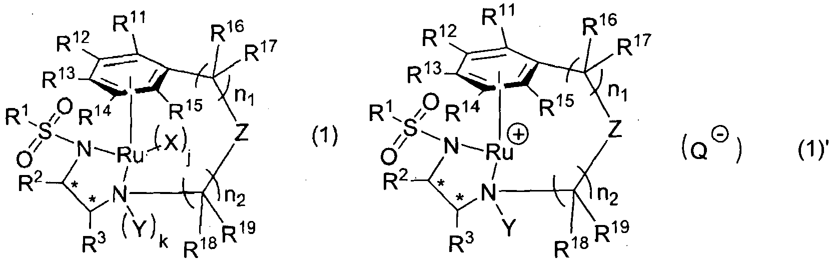 where
R¹ represents an alkyl group having 1 to 10 carbon atoms; a halogenated alkyl group having 1 to 10 carbon atoms; a 10-camphoryl group; an amino group which may be substituted with one or two alkyl groups having 1 to 10 carbon atoms; or an aryl group, provided that the aryl group may be substituted with one or more selected from alkyl groups having 1 to 10 carbon atoms, halogenated alkyl groups having 1 to 10 carbon atoms, halogen atoms, cyano groups (-CN), amino groups, alkylamino groups (-NR²¹R²¹), 5- or 6-membered cyclic amino groups, acylamino groups (-NH-CO-R²⁰), hydroxyl groups, alkoxy groups (-OR²⁰), acyl groups (-COR²⁰), carboxyl groups, alkoxycarbonyl groups (-COOR²⁰), phenoxycarbonyl groups, mercapto groups, alkylthio groups (-SR²⁰), silyl groups (-SiR²⁰R²¹R²²), and nitro groups (-NO₂),
R²⁰, R²¹, and R²² each independently represent a hydrogen atom, an alkyl group having 1 to 10 carbon atoms, or a cycloalkyl group having 3 to 10 carbon atoms,
Y represents a hydrogen atom,
X represents a trifluoromethanesulfonyloxy group, a p-toluenesulfonyloxy group, a methanesulfonyloxy group, a benzenesulfonyloxy group, a hydrogen atom, or a halogen atom,
Q^{Θ} represents a counter anion,
j and k each represent 0 or 1, provided that cases where j+k=1 are excluded, R² and R³ each independently represent a hydrogen atom; an alkyl group having 1 to 10 carbon atoms; a phenyl group (provided that the phenyl group may be substituted with one or more selected from alkyl groups having 1 to 10 carbon atoms, alkoxy groups having 1 to 10 carbon atoms, and halogen atoms); or a cycloalkyl group having 3 to 8 carbon atoms, or R² and R³ may together form a ring,
R¹¹, R¹², R¹³, R¹⁴, and R¹⁵ each independently represent a hydrogen atom, an alkyl group having 1 to 10 carbon atoms, or an alkoxy group having 1 to 10 carbon atoms,
R¹⁶, R¹⁷, R¹⁸_{,} and R¹⁹ each independently represent a hydrogen atom, a hydroxyl group, an alkyl group having 1 to 10 carbon atoms, or an alkoxy group having 1 to 10 carbon atoms, or R¹⁶, R¹⁷, and the carbon atom to which R¹⁶ and R¹⁷ are bonded and/or R¹⁸, R¹⁹, and the carbon atom to which R¹⁸ and R¹⁹ are bonded may form a carbonyl group(s),
Z represents an oxygen atom, a sulfur atom, or a methylene,
n₁ represents 1 or 2, and n₂ represents any integer of 1 to 3, and
each * indicates an asymmetric carbon atom, provided that when R² and/or
R³ is/are a hydrogen atom(s), the carbon atom to which the hydrogen atom is bonded is not an asymmetric carbon atom,
the β-keto-α-aminocarboxylic acid ester is represented by the following general formula (2): 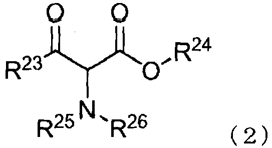 (where
R²³ represents a hydrocarbon group which has 11 to 21 carbon atoms and which may be substituted with one or more hydroxyl groups,
R²⁴ represents a hydrogen atom or a hydrocarbon group having 1 to 10 carbon atoms, and
R²⁵ and R²⁶, which may be the same or different, each represent a hydrogen atom, an alkyl group which has 1 to 10 carbon atoms and which may be substituted with one or more selected from halogen atoms and hydroxyl groups, an acyl group which has 1 to 24 carbon atoms and which may be substituted with one or more selected from halogen atoms and hydroxyl groups, or an amino-protecting group, or R²⁵ and R²⁶ may form, together with the adjacent nitrogen atom, a heterocycle which may be substituted with one or more hydroxyl groups), and
the optically active β-hydroxy-α-aminocarboxylic acid ester is represented by the following general formula (3) or (4): 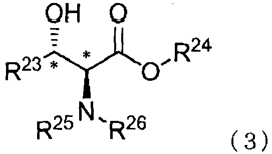 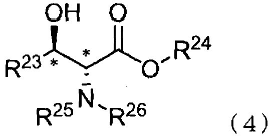 , where
each * indicates an asymmetric carbon atom, and
R²³, R²⁴, R²⁵, and R²⁶ are the same as those described above.

2. The production method according to claim 1, wherein formic acid is used as the hydrogen donor.

3. The production method according to claim 1 or 2, wherein the reaction is carried out in the coexistence of a base.

4. The production method according to any one of claims 1 to 3, wherein the base used for carrying out the asymmetric reduction reaction is an organic amine having 3 to 30 carbon atoms.

5. The production method according to any one of claims 1 to 4, wherein a major product of the optically active β-hydroxy-α-aminocarboxylic acid ester is a (2R,3R) isomer represented by general formula (4),
the formation ratio (2R,3R) isomer:(2S,3R) isomer is 85:15 to 100:0; and
the reaction is completed within a reaction time of 10 hours.

6. The production method according to any one of claims 1 to 4, wherein a major product of the optically active β-hydroxy-α-aminocarboxylic acid ester is a (2R,3R) isomer represented by general formula (4),
the formation ratio (2R,3R) isomer:(2S,3R) isomer is 85:15 to 100:0; and
the reaction is completed within a reaction time of 20 hours when the molar ratio of the ruthenium complex represented by general formula (1) to the β-keto-α-aminocarboxylic acid ester represented by general formula (2) is 1/500 to 1/10000.

7. The production method according to any one of claims 1 to 5, wherein the molar ratio of the ruthenium complex represented by general formula (1) to the β-keto-α-aminocarboxylic acid ester represented by general formula (2) is 1/250 to 1/10000.

8. The production method according to any one of claims 4 to 6, wherein at least one of triethylamine, tributylamine, and diisopropylethylamine is used as the organic amine.

## Patentansprüche

1. Verfahren zum Herstellen eines optisch aktiven β-hydroxy-α-Aminopolycarbonsäureesters, mit Durchführen einer asymmetrischen Reduktionsreaktion eines β-keto-α-Aminopolycarbonsäureesters in der Gegenwart eines Ruthenium-Komplexes und eines Wasserstoffdonors, wobei
der Ruthenium-Komplex durch die folgende generische Formel (1) oder (1)' wiedergegeben wird: 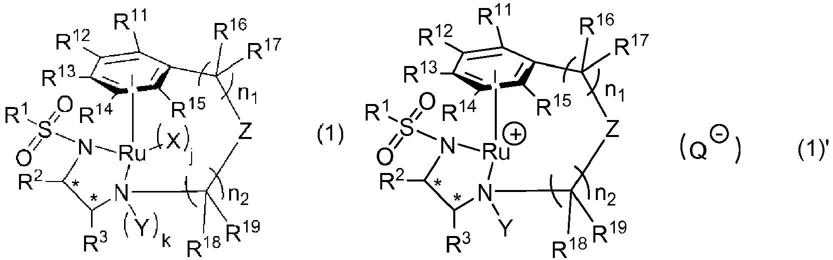
wobei R¹ eine Alkylgruppe mit 1 bis 10 Kohlenstoffatomen; eine halogenhaltige Alkylgruppe mit 1 bis 10 Kohlenstoffatomen; eine 10-Campherylgruppe; eine Aminogruppe, die mit einer oder zwei Alkylgruppen mit 1 bis 10 Kohlenstoffatomen substituiert sein kann; oder eine Alkylgruppe, unter der Berücksichtigung, dass die Alkylgruppe mit einem oder mehr substituiert sein kann, das ausgewählt ist aus einer Alkylgruppe mit 1 bis 10 Kohlenstoffatomen; einer halogenhaltige Alkylgruppe mit 1 bis 10 Kohlenstoffatomen, Halogenatomen, Cyanogruppen (-CN), Aminogruppen, Alkylaminogruppen (-NR²⁰R²¹) 5- oder 6-teilige zyklische Aminogruppen, Acylaminogruppen (-NH-CO-R²⁰), Hydroxylgruppen, Alkoxygruppen (-OR²⁰), Acylgruppen (-CO-R²⁰), Carboxylgruppen, Alkoxycarbylgruppen (-COOR²⁰), Phenoxycarbylgruppen, Mercaptogruppen, Alkylthiogruppen (-SR²⁰), Silylgruppen (-SiR²⁰R²¹R²²), und Nitrogruppen (-NO₂), darstellt,
R²⁰, R²¹ und R²² jeweils unabhängig ein Wasserstoffatom, eine Alkylgruppe mit 1 bis 10 Kohlenstoffatomen oder eine Cycloalkylgruppe mit 3 bis 10 Kohlenstoffatomen darstellen,
Y ein Wasserstoffatom darstellt,
X eine Trifluoromethanesulfonyloxygruppe, a p-Toluenesulfonyloxygruppe, eine Methanesulfonyloxygruppe, eine Benzenesulfonyloxygruppe, ein Wasserstoffatom oder ein Hallogenatom darstellt,
Q_{Θ} ein Gegenanion darstellt,
j und k jeweils 0 oder 1 darstellen, unter der Maßgabe, dass Fälle ausgeschlossen sind, in denen j+k=1,
R² und R³ jeweils unabhängig ein Wasserstoffatom; eine Alkylgruppe mit 1 bis 10 Kohlenstoffatomen; eine Phenylgruppe (unter der Maßgabe, dass die Phenylgruppe mit einem oder mehreren ausgewählt aus Alkylgruppen mit 1 bis 10 Kohlenstoffatomen, Alkoxygruppen mit 1 bis 10 Kohlenstoffatomen und Halogenatomen substituiert werden kann); oder eine Cycloalkylgruppe mit 3 bis 8 Kohlenstoffatomen darstellen, oder R² und R³ zusammen einen Ring bilden können,
R¹¹, R¹², R¹³, R¹⁴ und R¹⁵ jeweils unabhängig ein Wasserstoffatom, eine Alkylgruppe mit 1 bis 10 Kohlenstoffatomen oder eine Alkoxygruppe mit 1 bis 10 Kohlenstoffatomen darstellen,
R¹⁶, R¹⁷, R¹⁸ und R¹⁹ jeweils unabhängig ein Wasserstoffatom, eine Hydroxylgruppe, eine Alkylgruppe mit 1 bis 10 Kohlenstoffatomen oder eine Alkoxygruppe mit 1 bis 10 Kohlenstoffatomen darstellen, oder R¹⁶, R¹⁷ und das Kohlenstoffatom, mit dem R¹⁶ und R¹⁷ und/oder R¹⁸ und R¹⁹ verbunden sind, und das Kohlenstoffatom, mit dem R¹⁸ und R¹⁹ verbunden sind, (eine) Carbonylgruppe(n) bilden können,
Z eine Sauerstoffatom, ein Schwefelatom oder a Methylen darstellt,
n₁ für 1 oder 2 steht und n₂ für eine ganze Zahl von 1 bis 3 steht, und
jedes * ein asymmetrisches Kohlenstoffatom angibt, unter der Maßgabe, dass, wenn R² und/oder R³ ein (mehrere) Wasserstoffatom(e) ist/sind, das Kohlenstoffatom, mit dem das Wasserstoffatom verbunden ist, kein asymmetrisches Kohlenstoffatom ist,
wobei der β-keto-α-Aminopolycarbonsäureester durch die folgende generische Formel (2) wiedergegeben wird: 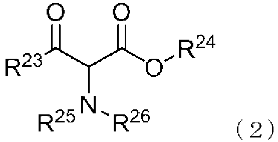 (wobei
R²³ eine Kohlenwasserstoffgruppe mit 11 bis 21 Kohlenstoffatomen darstellt und die mit einer oder mehr Hydroxylgruppen substituiert ist,
R²⁴ ein Wasserstoffatom oder eine Kohlenwasserstoffgruppe mit 1 bis 10 Kohlenstoffatomen darstellt, und
R²⁵ uns R²⁶, die das Gleiche oder etwas unterschiedliches sein können, jeweils eine Wasserstoffatom eine Alkylgruppe mit 1 bis 10 Kohlenstoffatomen und die mit einem oder mehreren ausgewählt aus Halogenatomen und Hydroxylgruppen, einer Acylgruppe mit 1 bis 24 Kohlenstoffatomen und die mit einem oder mehreren ausgewählt aus Halogenatomen und Hydroxylgruppen substituiert sein kann, oder eine Amino-schützenden Gruppe substituiert sein kann, oder R²⁵ uns R²⁶ können, zusammen mit den angrenzenden Stickstoffatomen, eine Heterocyclus bilden, der mit einer oder mehreren Hydroxylgruppen substituiert sein kann), und
der optisch aktive β-hydroxy-α-Aminopolycarbonsäureester durch die folgende generische Formel (3) oder (4) wiedergegeben wird: 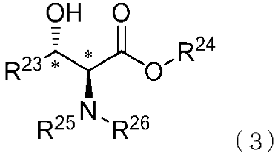 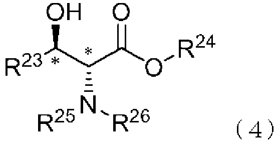 , wobei jedes * ein asymmetrisches Kohlenstoffatom darstellt, und
R²³, R²⁴, R²⁵, and R²⁶ die Selben wie oben beschrieben sind.

2. Herstellverfahren nach Anspruch 1, wobei Ameisensäure als Wasserstoffdonor verwendet wird.

3. Herstellverfahren nach Anspruch 1 oder 2, wobei die Reaktion in Koexistenz einer Base durchgeführt wird.

4. Herstellverfahren nach einem der Ansprüche 1 bis 3, wobei die zum Ausführen der asymmetrischen Redoxreaktion verwendete Base ein organisches Amin mit 3 bis 30 Kohlenstoffatomen ist.

5. Herstellverfahren nach einem der Ansprüche 1 bis 4, wobei ein Hauptprodukt des optisch aktiven β-hydroxy-α-Aminopolycarbonsäureesters ein (2R,3R) Isomer ist, das durch die generische Formel (4) wiedergeben wird,
wobei das Bildungsverhältnis (2R,3R) Isomer : (2S,3R) Isomer 85:15 bis 100:0 ist, und
die Reaktion innerhalb einer Reaktionszeit von 10 Stunden abgeschlossen ist.

6. Herstellverfahren nach einem der Ansprüche 1 bis 4, wobei ein Hauptprodukt des optisch aktiven β-hydroxy-α-Aminopolycarbonsäureesters ein (2R,3R) Isomer ist, das durch die generische Formel (4) wiedergeben wird,
wobei das Bildungsverhältnis (2R,3R) Isomer : (2S,3R) Isomer 85:15 bis 100:0 ist, und
die Reaktion innerhalb einer Reaktionszeit von 20 Stunden abgeschlossen ist, wenn das Molverhältnis des Rutheniumkomplexes, der durch die generische Formel (1) wiedergegeben wird, zu dem β-keto-α-Aminopolycarbonsäureester, der durch die generische Formel (2) wiedergegeben wird, 1/500 bis 1/10000 ist.

7. Herstellverfahren nach einem der Ansprüche 1 bis 5, wobei das Molverhältnis des Rutheniumkomplexes, der durch die generische Formel (1) wiedergegeben wird, zu dem β-keto-α-Aminopolycarbonsäureester, der durch die generische Formel (2) wiedergegeben wird, 1/250 bis 1/10000 ist.

8. Herstellverfahren nach einem der Ansprüche 4 bis 6, wobei zumindest eins aus Triethylamin, Tributylamin und Diisopropylethylamin als das organische Amid verwendet wird.

## Revendications

1. Procédé de préparation d'un ester d'acide β-hydroxy-α-amino-carboxylique optiquement actif, comprenant la réalisation d'une réaction de réduction asymétrique d'un ester d'acide β-céto-α-amino-carboxylique en présence d'un complexe de ruthénium et d'un donneur d'hydrogène, dans lequel procédé :
le complexe de ruthénium est représenté par la formule générale (1) ou (1') suivante : 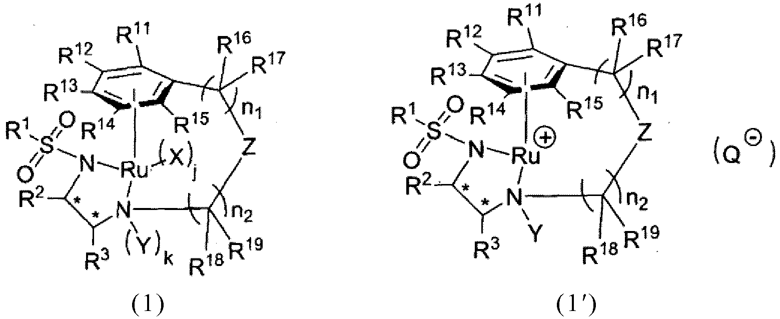 dans lesquelles formules
- R¹ représente un groupe alkyle comportant 1 à 10 atomes de carbone, un groupe alkyle halogéné comportant 1 à 10 atomes de carbone, un groupe camphor-10-yle, un groupe amino qui peut porter un ou deux substituant(s) alkyle comportant 1 à 10 atomes de carbone,
ou un groupe aryle,
étant entendu que ce groupe aryle peut porter un ou plusieurs substituant(s) choisi(s) parmi les groupes alkyle comportant 1 à 10 atomes de carbone, les groupes alkyle halogénés comportant 1 à 10 atomes de carbone, les atomes d'halogène, et les groupes cyano (-CN), amino, alkyl-amino (-NR²⁰R²¹), amino cycliques à 5 ou 6 chaînons, acyl-amino (-NH-CO-R²⁰) , hydroxyle, alcoxy (-OR²⁰), acyle (-CO-R²⁰), carboxyle, alcoxy-carbonyle (-CO-OR²⁰), phénoxy-carbonyle, sulfhydryle, alkylthio (-SR²⁰), silyle (-SiR²⁰R²¹R²²) et nitro (-NO₂), où R²⁰, R²¹ et R²² représentent chacun, indépendamment, un atome d'hydrogène, un groupe alkyle comportant 1 à 10 atomes de carbone, ou un groupe cycloalkyle comportant 3 à 10 atomes de carbone,
- Y représente un atome d'hydrogène,
- X représente un groupe trifluoro-méthanesulfonyl-oxy, para-toluène-sulfonyl-oxy, méthanesulfonyl-oxy ou benzènesulfonyl-oxy, ou un atome d'hydrogène ou d'halogène,
- Q^{Θ} représente un contre-ion,
- les indices j et k valent chacun 0 ou 1, sous réserve qu'il soit exclu que j+k vaille 1,
- R² et R³ représentent chacun, indépendamment, un atome d'hydrogène, un groupe alkyle comportant 1 à 10 atomes de carbone,
un groupe phényle, étant entendu que ce groupe phényle peut porter un ou plusieurs substituant(s) choisi(s) parmi les groupes alkyle comportant 1 à 10 atomes de carbone, les groupes alcoxy comportant 1 à 10 atomes de carbone et les atomes d'halogène, ou un groupe cycloalkyle comportant 3 à 8 atomes de carbone, ou bien R² et R³ représentent des entités formant conjointement un cycle,
- R¹¹, R¹², R¹³, R¹⁴ et R¹⁵ représentent chacun, indépendamment, un atome d'hydrogène, un groupe alkyle comportant 1 à 10 atomes de carbone ou un groupe alcoxy comportant 1 à 10 atomes de carbone,
- R¹⁶, R¹⁷ R¹⁸ et R¹⁹ représentent chacun, indépendamment, un atome d'hydrogène, un groupe hydroxyle, un groupe alkyle comportant 1 à 10 atomes de carbone ou un groupe alcoxy comportant 1 à 10 atomes de carbone, ou bien les entités symbolisées par R¹⁶ et R¹⁷ et l'atome de carbone auquel sont liées ces entités R¹⁶ et R¹⁷ et/ou les entités symbolisées par R¹⁸ et R¹⁹ et l'atome de carbone auquel sont liées ces entités R¹⁸ et R¹⁹ constituent un ou des groupe(s) carbonyle,
- Z représente un atome d'oxygène, un atome de soufre, ou un groupe méthanediyle,
- l'indice n₁ vaut 1 ou 2, et l'indice n₂ est un nombre entier valant de 1 à 3,
- et chaque astérisque * indique un atome de carbone asymétrique, étant entendu que si R² et/ou R³ représente(nt) un ou des atome(s) d'hydrogène, l'atome de carbone auquel est lié un tel atome d'hydrogène n'est pas un atome de carbone asymétrique ;
l'ester d'acide β-céto-α-amino-carboxylique est représenté par la formule générale (2) suivante : 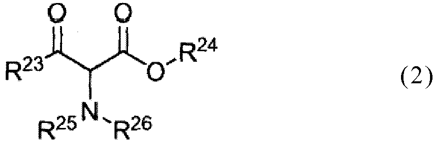 dans laquelle
- R²³ représente un groupe hydrocarboné comportant de 11 à 21 atomes de carbone, qui peut porter un ou plusieurs substituant(s) hydroxy,
- R²⁴ représente un atome d'hydrogène ou un groupe hydrocarboné comportant 1 à 10 atomes de carbone,
- et R²⁵ et R²⁶ représentent des entités qui peuvent être identiques ou différentes et représentent chacun un atome d'hydrogène, un groupe alkyle comportant 1 à 10 atomes de carbone, qui peut porter un ou plusieurs substituant(s) choisi(s) parmi des atomes d'halogène et des groupes hydroxyle, un groupe acyle comportant 1 à 24 atomes de carbone, qui peut porter un ou plusieurs substituant(s) choisi(s) parmi des atomes d'halogène et des groupes hydroxyle, ou un groupe amino-protecteur, ou bien R²⁵ et R²⁶ représentent des entités qui forment, conjointement avec l'atome d'azote adjacent, un hétérocycle qui peut porter un ou plusieurs substituant(s) hydroxy ;
et l'ester d'acide β-hydroxy-α-amino-carboxylique optiquement actif est représenté par la formule générale (3) ou (4) suivante : 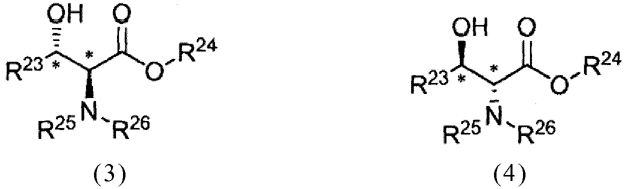 dans lesquelles formules
- chaque astérisque * indique un atome de carbone asymétrique,
- et les symboles R²³, R²⁴, R²⁵ et R²⁶ ont les mêmes significations que celles données ci-dessus.

2. Procédé de préparation conforme à la revendication 1, dans lequel on utilise de l'acide formique en tant que donneur d'hydrogène.

3. Procédé de préparation conforme à la revendication 1 ou 2, dans lequel la réaction est réalisée en présence d'une base.

4. Procédé de préparation conforme à l'une des revendications 1 à 3, dans lequel la base utilisée pour réaliser la réaction de réduction asymétrique est une amine organique comportant de 3 à 30 atomes de carbone.

5. Procédé de préparation conforme à l'une des revendications 1 à 4, dans lequel
- le produit majoritaire dans l'ester d'acide β-hydroxy-α-amino-carboxylique optiquement actif est l'isomère (2R,3R) représenté par la formule générale (4),
- le rapport de formation des isomères (2R,3R)/2S,3R) vaut de 85/15 à 100/0,
- et la réaction est achevée en un temps de réaction de moins de 10 heures.

6. Procédé de préparation conforme à l'une des revendications 1 à 4, dans lequel
- le produit majoritaire dans l'ester d'acide β-hydroxy-α-amino-carboxylique optiquement actif est l'isomère (2R,3R) représenté par la formule générale (4),
- le rapport de formation des isomères (2R,3R)/2S,3R) vaut de 85/15 à 100/0,
- et la réaction est achevée en un temps de réaction de moins de 20 heures quand le rapport molaire du complexe de ruthénium représenté par la formule générale (1) à l'ester d'acide β-céto-α-amino-carboxylique représenté par la formule générale (2) vaut de 1/500 à 1/10000.

7. Procédé de préparation conforme à l'une des revendications 1 à 5, dans lequel le rapport molaire du complexe de ruthénium de formule générale (1) à l'ester d'acide β-céto-α-amino-carboxylique de formule générale (2) vaut de 1/250 à 1/10000.

8. Procédé de préparation conforme à l'une des revendications 4 à 6, dans lequel on utilise en tant qu'amine organique au moins l'une des suivantes : triéthyl-amine, tributyl-amine et diisopropyl-éthyl-amine.
